(19) Europäisches Patentamt / European Patent Office / Office européen des brevets

(11) **EP 3 807 287 B1**

(12) **EUROPEAN PATENT SPECIFICATION**

(45) Date of publication and mention
of the grant of the patent:
**30.07.2025 Bulletin 2025/31**

(21) Application number: **19736980.4**

(22) Date of filing: **04.06.2019**

(51) International Patent Classification (IPC):
*C07F 7/28* (2006.01)  *A61K 8/35* (2006.01)
*C07C 49/255* (2006.01)  *A61K 8/19* (2006.01)
*A61K 8/29* (2006.01)  *A61Q 1/02* (2006.01)
*A61Q 5/00* (2006.01)  *A61Q 17/04* (2006.01)
*A61Q 19/00* (2006.01)  *C07C 45/77* (2006.01)
*A61Q 19/08* (2006.01)

(52) Cooperative Patent Classification (CPC):
(C-Sets available)
**C07F 7/28; A61K 8/19; A61K 8/29; A61K 8/35;
A61Q 1/02; A61Q 5/00; A61Q 17/04; A61Q 19/00;
A61Q 19/08; C07C 45/77;** A61K 2800/58;
A61K 2800/805                    (Cont.)

(86) International application number:
**PCT/EP2019/000174**

(87) International publication number:
**WO 2019/238261 (19.12.2019 Gazette 2019/51)**

(54) **METAL COMPLEXES OF BETA-DIKETONES AND/OR POLYPHENOLS BY GREEN CHEMISTRY, PREPARATION METHOD THEREOF, SUNSCREEN THEREOF, SKIN OR HAIR TONE CONCEALER THEREOF, HAIR DYEING THEREOF AND OTHER USES THEREOF**

METALLKOMPLEXE VON BETA-DIKETONEN UND / ODER POLYPHENOLEN DURCH GRÜNE CHEMIE, VERFAHREN ZU IHRER HERSTELLUNG, SONNENSCHUTZMITTEL, HAUT- ODER HAARTONABDECKER DARAUS, HAARFÄRBUNG DARAUS UND ANDERE VERWENDUNGEN DERSELBEN

COMPLEXES MÉTALLIQUES DE BETA-DICÉTONES ET/OU DE POLYPHÉNOLS PAR CHIMIE VERTE, PROCÉDÉ DE PRÉPARATION, ÉCRAN SOLAIRE, CORRECTEUR DE TEINT DE LA PEAU OU DES CHEVEUX, TEINTURE CAPILLAIRE ET UTILISATIONS ASSOCIÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.06.2018 EP 18075007
08.05.2019 EP 19075006**

(43) Date of publication of application:
**21.04.2021 Bulletin 2021/16**

(73) Proprietor: **RAMIREZ RIOS, Liliana Patricia
2565LH Den Haag (NL)**

(72) Inventor: **RAMIREZ RIOS, Liliana Patricia
2565LH Den Haag (NL)**

(56) References cited:
WO-A1-2006/106366    CN-A- 107 417 719
US-A- 4 263 333      US-A1- 2008 254 216
US-A1- 2011 250 126  US-A1- 2017 275 309

• **VIGATO P A ET AL: "The evolution of @b-diketone or @b-diketophenol ligands and related complexes", COORDINATION CHEMISTRY REVIEWS, ELSEVIER SCIENCE, AMSTERDAM, NL, vol. 253, no. 7-8, 1 April 2009 (2009-04-01), pages 1099 - 1201, XP026004375, ISSN: 0010-8545, [retrieved on 20080730], DOI: 10.1016/ J.CCR.2008.07.013**

- **ANA M. LÓPEZ-PERIAGO ET AL: "Metal-Organic Frameworks Precipitated by Reactive Crystallization in Supercritical CO 2", CRYSTAL GROWTH & DESIGN., vol. 17, no. 5, 3 May 2017 (2017-05-03), US, pages 2864 - 2872, XP055625943, ISSN: 1528-7483, DOI: 10.1021/ acs.cgd.7b00378**
- **M.M RAHMAN ET AL: "Optical properties and X-ray photoelectron spectroscopic study of pure and Pb-doped TiO 2 thin films", JOURNAL OF PHYSICS AND CHEMISTRY OF SOLIDS., vol. 60, no. 2, 1 January 1999 (1999-01-01), GB, pages 201 - 210, XP055626042, ISSN: 0022-3697, DOI: 10.1016/S0022-3697(98)00264-9**
- **SEVASTYANOV V G ET AL: "Low-temperature synthesis of nanodispersed titanium, zirconium, and hafnium carbides", RUSSIAN JOURNAL OF INORGANIC CHEMISTRY, NAUKA/INTERPERIODICA, MO, vol. 56, no. 5, 1 June 2011 (2011-06-01), pages 661 - 672, XP019911339, ISSN: 1531-8613, DOI: 10.1134/S0036023611050214**

Remarks:

The file contains technical information submitted after the application was filed and not included in this specification

(52) Cooperative Patent Classification (CPC): (Cont.)

C-Sets
**C07C 45/77, C07C 49/255;**
**C07C 45/77, C07C 49/92**

## Description

### Field of the invention

Metal complexes of polyphenols or of metal complexes of β-diketones and polyphenols by green chemistry, preparation method thereof, sunscreen thereof, skin or hair tone concealer thereof, hair dyeing thereof and other uses thereof.

The present invention relates to a method to synthesize with high yield a colored metal complex of polyphenols or of a highly coloured metal complex of β-diketones and polyphenols by mechanochemistry without using aqueous or organic solvents and covers new complexes obtained therefrom. In an embodiment, only the reactants, a metal alkoxide and a polyphenol or a metal alkoxide and a β-diketone and a polyphenol, are present and react at different molar ratios to form a metal complex in the form of a homogeneous colored material (i.e. powder) with high yield. If curcumin is used, the color of the final metal complex dye depends on several factors such as the curcuminoids, the metal, the type of alkoxide used, the stoichiometric molar ratio of both reactants and the additives used. If titanium alkoxide and any additive is used, a red or violet colored titanium curcumin complex in powder form is produced. The production process of the metal complex in powder form is characterized by facility of scale up. The entire product is ready to be used in several applications. If polyphenols such as ferulic acid, quercetin, ellagic acid or lignin are used instead of curcumin, other colored complexes are obtained using mechanochemistry. In addition, the product comprising metal complexes of polyphenols or of β-diketones and polyphenols is characterized by green chemistry manufacture. Besides, these metal complexes of polyphenols or of β-diketones and polyphenols in several formulations are characterized by improved stability under storage conditions and improved sun protection against UV rays for human skin and human hair. The product is ready to be used alone or in combination with other additives or active ingredients in different formulations. The invention also covers the use of these metal β-diketones/polyphenol complexes in food, cosmetics, the pharmaceutical field and in the creativity field, as sunscreen, skin and hair concealer or foundation in powder form or cream, keratinous dye, textile dye, food dye, dye emulsions, miniemulsions, polymer colloids and catalysts.

The production process of these novel metal-complex dyes is also characterized by rapid production of colored compounds without using solvents and, thus, ecofriendliness. The process of the production of metal β-diketone and/or polyphenol complexes is characterized by the non-formation of toxic by-products and high yield.

In addition, the present invention comprises a hair dyeing molecule or formulation and procedure combining excellent dyeing properties with reduced risk of development cancer or allergies, while being ecological and of the natural origin.

The present invention provides a process for the synthesis of metal complexes of polyphenols or of β-diketones (e.g., curcumin) and polyphenols (e.g., ferulic acid, quercetin, ellagic acid, lignin) by mechanochemistry and a food, a cosmetic, a pharmaceutical or a paint formulation thereof and other uses thereof. These formulations are colored and exhibit an excellent stability in the form of powder or colloids. In particular, the titanium curcumin complexes are reddish-purple colored; the titanium ferulic acid complexes are orange-yellow; the titanium quercetin complexes are reddish-brown; the titanium ellagic acid are orange; and titanium lignin complexes are yellow. The color palette is very wide and varies with changes in the reactants and in the final formulation.

Small changes in the formulation can produce a marked difference in color such as violet, green, brown or black. The sunscreen formulations with concealer effect using the metal complex dyes of the present invention are stable and do not fade. The present invention provides also a temporal and semipermanent hair dye and a hair dyeing process with excellent dyeing properties.

### Background of the invention

Curcumin and the curcuminoids are both naturally occurring β-diketones and polyphenols. Curcumin (synonyms: CI natural yellow 3, CI 75300, E100i,ii,iii, Diferuloylmethane, turmeric yellow) is a naturally occurring diarylheptanoid isolated from Zingiberaceae.

Chemical names:

E100 i: 1,7-Bis(4-hidroxy-3-methoxyphenyl)hepta-1,6-diene-3,5-dione
E100 ii: 1-(4-Hydroxyphenyl)-7-(4-hydroxy-3-methoxy-phenyl-)hepta-1,6-diene-3,5-dione
E100 iii: 1-7-Bis(4-hydroxyphenyl)hepta-1,6-diene-3,5-dione
Curcumin has low intrinsic toxicity and is "Generally Recognized as Safe (GRAS)" by the Food and Drug Administration (FDA), European Union Herbal Monograph on Curcuma longa L, rhizoma (EMA/HMPC/329755/2017)
Curcumin is an oil-soluble pigment. According to FAO (2004) in *Curcumin chemical and technical assessment,* 61st JECFA., curcumin is almost insoluble in water under acidic and neutral pH conditions and soluble in alkali medium. At pH<1, aqueous solutions of curcumin present a red color that indicates the protonate form. In the pH range between 1 and 7, curcumin is in neutral form and the solutions thereof are yellow. At pH>7.5, the color changes to red.

Several medicinal and therapeutic effects have been reported or passed from mouth to mouth since ancient times. Nowadays, there is a quest for medicinal research regarding curcumin due to its well-known or unknown benefits for human health. Curcumin has potent anti-inflammatory properties. However, less effort has been made to find solutions for already known problems within the curcumin chemistry, such as low solubility in aqueous solutions and most common solvents, poor bioavailability, low resistance to fading and pH-depending degradation, i.e. rapid hydrolytic degradation at physiological pHs that does not facilitate their therapeutically use.

One of the objects of the present invention is to provide a green synthesis of metal curcumin complexes which enhance the solubility in different media and can overcome the poor bioavailability due to the further encapsulation in polymer particles, which can provide longer blood circulation. Another purpose of the complexation of curcumin with a metal (e.g. titanium or zinc or both) is the enhancement of the properties of both components, such as the UV shielding effect, antimicrobial, anti-dandruff, anti-acne. Another purpose of the present invention is to produce a coloring mater such as red, violet, green, brown and others with healthy properties with less harmful reactants.

Curcumin is similar to the simplest $\beta$-diketone, acetylacetone, in the sense that it shows keto-enol tautomerism. Curcumin exists in equilibrium with its enol tautomer. However, both unsaturated phenolic groups attached to the acetylacetone central moiety generate a conjugated and flat structure that prones curcumin to react. Besides, the phenolic OH groups are another moiety for reacting.

Wanninger[1] made a detailed description of the metal curcumin complexes and highlighted the advantages of complexation of the curcumin, such as the increase of bioavailability. He also discussed some of the problems commonly found in metal curcumin chemistry, such as low crystallinity and low solubility in water and common solvents. As a result, the clarification of the structure by x-ray diffraction is rare in the literature. This problem is even more pronounced in homoleptic curcumin.

The present invention resolves the solubility or dispersibility in organic and aqueous media, among other aspects already mentioned before.

Beck and co-workers[2] developed a process to synthetize several metal complexes of curcumin, which is used as a general protocol of metal curcumin complexes. In brief, after a deprotonation of curcumin suspension in methanol or dichloromethane with a base and a metal chloride, different metal curcumin complexes were obtained in solution after 6 hours of synthesis.

Another approach is blocking the OH groups at the fourth position of the phenyl rings. This was originally carried out by Spicer and Strickland[3] by studying the Rosocyanin (discussed below), but no changes in the product were observed.

A further approach was the synthesis of heteroleptic curcumin containing other molecules that act as spectator ligands. The method of preparation of Rubrocurcumin (discussed below) belongs to this approach where oxalic acid was used.

Furthermore, a well-known approach was the synthesis via the $\beta$-diketone moiety with boronic acid or boronic acid derivatives to produce boron curcumin derivatives such as rosocyanin and rubrocurcumin (see below).

**Metal curcumin dye, red curcumin**

The red boron-curcumin complex has been the first metal curcumin complex to be discovered and the most widely studied. It highlighted the reaction of curcumin with boric acid due to the $\beta$-diketone moiety.

The well-known red dye product of the reaction of boric acid (or boronic acid derivatives) with curcumin is used for the spectrophotometric detection of boron.

Two boron-curcumin complexes are known, Rosocyanin[3] and Rubrocurcumin,[4] without and with oxalate moieties, respectively. Rosocyanin is a red colored complex more stable against hydrolysis and more sensitive to boron determination. Rosocyanin is formed in strongly acid conditions to protonate the curcumin in almost water-free solution, since the presence of water inhibits the formation of both boron curcumin complexes. Rosocyanin has a crimson-red color in acid condition and changes to blue-green in a basic medium. The process of formation of boron-curcumin complexes implicates the evaporation of a significant amount of the solvents under carefully controlled conditions.

Hayes and Metcalfe[5] improved the synthesis of boron-curcumin complexes by non-aqueous medium without evaporation of the reactants. A given amount of aqueous solution of boric acid (or methyl borate in methanol) and an amount of sodium hydroxide solution was heated to dryness. To this powder, a given amount of solution of curcumin in glacial acetic acid (0.125%) and the same amount of 1:1 solution of concentrated sulfuric acid and glacial acetic acid was added. Thus, the boron-curcumin complex was formed in solution and redissolved in methanol. The final complex contained one boron atom and three curcumin ligands. Only nitrate and fluoride ions generated interferences in the spectrophotometric measurements.

The process still used concentrated mineral and organic acids that made the process very health-risky and introduced more steps to get the final complex free of impurities.

Tsuchikawa et al.[6] synthetized a complex of curcumin, catechol derivatives and boron-n-butoxide under refluxing toluene between three and four hours. Thus, a similar red colored compound to Rubrocurcumin was obtained using solution-based synthesis. Again, this synthesis used a large amount of solvents, the elimination of which makes it a time-consuming process.

The boron-curcumin complexes have been widely studied and similar compounds are obtained replacing the oxalic acid by catechol, as a.m., or citric acid. Besides, several boronic acid derivatives have also been used with similar final complexation.

**Silica curcumin**

Another strategy uses the sol-gel synthesis to produce silica-curcumin gels. Almost any nanostructured material can be tailor-made from the sol-gel synthesis by variation of parameters, such as type and amount of precursor, chelating agent, solvents, catalyst, temperature, pH or calcination temperature.

Das[7] synthetized silica-curcumin by sol-gel using tetraethyl orthosilicate (TEOS) as a precursor. Curcumin previously dissolved in tetrahydrofuran (THF) was added to a aqueous solution of hydrochloric acid (HCl). To this mixture, TEOS was added dropwise at temperatures higher than 36°C and under nitrogen. The sample became dark red. After refluxing for 4 hours, gelation for 8 hours and drying at 70°C for other 8 hours, a glassy sample was obtained. The sample was ground for further analysis.

This previous process used an organic solvent for dissolving the curcumin and an aqueous acid solution to precipitate the silica particles into the curcumin. More than twenty hours were needed to synthesize the gel. It can be seen that curcumin from the previous process changes to red color in acid conditions and fades or degrades soon due its instability in acidic and alkaline conditions.

Non-aqueous sol-gel processes or non-hydrolytic sol-gel can be advantageous to produce hybrid materials due to the lower sensitivity to reaction conditions. In the present invention, no organic or aqueous solvent is used. The metal alkoxide and the curcumin compound are allowed to react by a non-aqueous sol-gel process. In the present invention, a metal curcumin complex is formed by the organic and aqueous solvent-free reaction of curcumin with a metal alkoxide at room temperature and by mechanochemistry. The metal precursor is a metal alkoxide, and the β-diketone chelating agent is preferably curcumin. Water, if present, is from the atmosphere or generated during the reaction. Water or the parental alcohol from the alkoxide is supposed to be expelled during the reaction, but the metal curcumin complex obtained in this invention is a homogeneously colored powder without precipitation or formation of two phases. The process is, presumably, a supramolecular arrangement of the molecules where either water or the parental alcohol are included in the supramolecular structure.

Oxygen atoms are provided by curcumin or by the metal alkoxide.

The metal alkoxide, and in particular titanium alkoxide, act as a catalyst and as a precursor of the sol-gel reaction.

The reaction is at room temperature, and only hand or mechanic maceration is required. Optionally, a moderate increase of the temperature is needed to reach the temperatures required in mechanochemistry.

**Sol-gel process from a β-diketone and a metal alkoxide**

The reaction of a β-diketone with a metal alkoxide[8] may proceed via four different modes of bonding of ligand moieties to metal atoms: oxygen-bonded; carbon-bonded; both carbon-bonded and oxygen-bonded; olefin-bonded.

Bradley et al.[9] disclosed the different synthetic route for the preparation of metal oxygen-bonded β-diketonate complexes from the reaction of β-diketone with metal alkoxides.

Metal or non-metal alkoxides react very readily or conveniently with the hydroxyl group of the β-diketone, liberating the parent alcohol that can be distilled as azeotropes with solvents like benzene. Thus:

$$M(OR)_x + mAH \xrightarrow{Benzene/reflux} M(A)_m(OR)_{x-m} + mROH$$

where $M(OR)_x$ is the metal alkoxide and A is the β-ketoenolate anion of the β-diketone.

β-ketoenolate anion may function as ligand to form oxygen-bonded β-diketonate complexes. However, other modes of bonding may also occur. There is no consensus on the selectivity for one type of bonding or for the formation of monomers and oligomers as a product of the reaction. Changing the metal and the alkyl chain of the metal alkoxide and the β-diketonate ligand of the β-diketone generates prominent differences in the product obtained.

Mehrota[8] described the synthesis of metal β-diketonates from metal alkoxide in solution-based reactions. In those reactions, solvent is used in huge quantities to let the reactants react or boil under reflux conditions at higher temperatures in order to push the reaction to completion by continuous fractionation of the alcohol liberated in the process.

Patent application DE000001945303A[10] disclosed the simultaneous production of titanium (III) β-diketonate complexes and an alkali alcoholate from the reaction of titanium (IV) alkoxide and β-diketone in presence of alkali amalgam under solvent reflux. It claimed to resolve the problem of the continuous distillation out of the parental alcohol produced, since the alkali alcoholate was precipited from the solution. An alkali amalgam was used as a reduction agent to facilitate the completion of the reaction. However, the process still used an organic solvent, such as benzene or toluene, in which the metal β-diketone is soluble but the alkali alcoholate is insoluble. The catalyst used, an alloy of sodium and mercury, is very hazardous.

US 2008/0254216 A1 discloses a metal complex compound comprising a β-diketonato ligand having an alkoxyalkyl-methyl group, and a method for preparing a metal-containing thin film using the metal complex compound by chemical vapor deposition (CVD) method.

It is well known that solvents can generate artifacts that hinder or promote the interaction of the reactants and hinder the synthesis of the desired product. The present invention of the synthesis of metal β-diketonate complexes from the chemomechanical reaction of a metal alkoxide and a β-diketone (e.g. curcumin) is characterized by a solvent-free and direct synthesis of complexes in powder form.

Hence, no artifacts are created by the use of solvents within the reaction that alter the interaction of the reactants. The present invention is also characterized by an economically attractive and straightforward implementation of large scale production. The most remarkable advantage of the present invention is that it fulfils all requirements to be a green synthesis.

## Reaction of metal (or non-metal) alkoxides with phenolic compounds

Alkoxy derivatives of elements tend to react with all hydroxy compounds, resulting in the replacement of their alkoxy groups. Thus, the reactions of alkoxides with alcohols, as well as phenols, generally called alcoholysis or alcohol interchange reactions are depending upon many factors such as the bulkiness of the alcohol or alkoxide moiety, the solubility, the fractionation of more volatile products, the fractionation in the presence of an inert solvent. Again, the use of a solvent such as benzene was highly recommended in order to complete the reaction (Chapter 2.4 in *metal alkoxide* by Bradley et al., 1978). As mentioned for β-diketonates, the alcohol interchange reaction is to be carried out in the presence of a solvent such as benzene. If no inert solvent is added, the reactants are left for days to be able to obtain a product, if any, and the yield is very low.

As mentioned above, the phenol groups of the curcumin may undergo a reaction with the metal alkoxide. In similar ways, polyphenols typically react with metal alkoxides to produce phenoxides releasing alcohols. Almost all phenolic compounds can act as antioxidants/prooxidants and can act as metal chelators.

## Mechanochemistry

Mechanochemistry is the chemistry that studies the chemical behavior of materials by mechanical effects without solvents or with negligible amounts of solvent in comparison with solution-based methods. The triboelectric effect can be achieved by hand using mortar and pestle, by a mechanochemical reactor, by mill reactor such as ball, vibratory, planetary.

EP2421509B1[11] disclosed methods for producing particles of a biologically active material dispersed in an at least partially milled grinding material using dry milling process at or above a volume fraction of 25 v/v% and with a particle size of less than 2000 nm. The biologically active material, among them a natural occurring material such as curcumin, was dry-milled by using a grinding matrix and/or a milling aid.

In the abovementioned EP2421509B1 patent, the curcumin and the grinding matrix are allowed to interact by mechanochemical method. None of the compounds referred to in the list of grinding matrixes or milling aids or facilitating agents is a metal alkoxide or derivative of metal alkoxide. In fact, the term grinding matrix was defined as any inert substance with which a biologically active material is or can be combined and milled. Thus, there is no possibility for a chemical reaction between the curcumin and other compounds present in that invention. There is no chance of formation of a complex between the curcumin and a metal alkoxide, since no metal alkoxide or derivatives of metal alkoxide were used in the abovementioned dry-milling.

In the present invention, the choice of the compounds is such that, in addition of the interaction by mechanochemistry, a chemical reaction is promoted, since both reactants - a polyphenol or a β-diketone and a polyphenol (e.g. curcumin) and a metal alkoxide-- are prone to form complexes by an oxygen-bonded β-diketonate route. In addition, only two components are present in the mill for the mechanochemical synthesis, and, optionally, further additives can be used depending on the further use.

The minimal use of solvent transforms the mechanochemistry into a green alternative which is sustainable, environment-friendly and cost-effective.

In the present invention, the metal complex formation is promoted by both a mechanochemical reaction route and an oxygen-bonded β-diketonate complexation route of two reactants: a β-diketone and a metal alkoxide. In contrast to prior applications of mechanochemistry, the present invention is effective in the sense that it delivers products with high homogeneity and stability and smaller amounts of additives or hazardous by-products.

**Lignin**

Lignin is a main component of lignocellulose biomass (around 30%) and the only renewable aromatic compound in nature. Lignin is highly branched aromatic polymeric phenol that is obtained as by-product from the pulp and paper-making and biofuel industries. Lignin is the main chromophore of wood and can be isolated from wood and pulp by mechanochemistry and subsequent chemical or biochemical processes. To date, mechanochemistry has been used in mechanical and high yield pulps to modify the natural native lignin, the so-called milled wood lignin, for defibrillation. In this way, the lignin is retained in the pulp together with hemicellulose and cellulose. These pulps are further bleached to remove the chromophores. Since lignin is still in the pulp, the brightness gained by bleaching is only temporal. By contrast, chemical pulps for separating lignin from hemicellulose and cellulose generally are based on aggressive chemicals and hence transform the original lignin from plants in a lignin with reduced reactivity, and thereby hindering the further use. However, the absence of lignin in the bleached chemical pulps does not eliminate the susceptibility of the bleached products to yellow.

**The undesired discoloration**

Polyphenols are often used as antioxidants or radical scavengers to protect products from discoloration. However, polyphenols suffer from color instability and are also prone to discoloration.

Lignin as well as curcumin are not exempt of these drawbacks and hence their reduced possibilities of industrial applications. Brightness or color reversion also known as yellowing, discoloration, or aging of pulp and paper products can be caused by several factors such as exposure to daylight, indoor illumination, heat, and air pollution.

**Lignin as a colorant**

Although light-induced discoloration of cellulosic pulps and paper products is one of the big chemistry problems that the wood industry has faced since the beginning, its mechanism and permanent solution are still an issue. Research in chemistry of light-induced yellowing generally involves the irradiation of simple model lignin compounds or model compounds in solution where the type of solvent might be relevant for the final results. Often, the results are inconsistent if applied to lignin.

Not only the study of the chemistry of the light induced yellowing of the lignin suffers from this drawback but also the extension of the applicability of lignin compounds in material science to produce other value-added materials. So far, a small fraction of lignin from the paper industry is used as dispersant agents, emulsifying agents, phenolic resins, polyurethane foams, and vanillin production. The biggest part of the lignin is waste or is recycled to produce energy for the processes of the pulp and paper industry. Thus, the potential of lignin to be used as a valuable product has not been developed. There is not prior work concerning the process and use of lignin as a residue of the paper industry to produce stable coloring matters.

In brief, polyphenols are rich in healthy properties but also susceptible to oxidation, degradation or discoloration.

Parisi, O. I.[12] et al. (2013) described some strategies to overcome the poor bioavailability and poor stability of the polyphenols to heat, light, moisture, oxygen, and pH. They highlighted the most important techniques of encapsulation, to prevent those drawbacks such as spray-drying, encapsulation using supercritical fluids, coacervation, emulsions, vesicles, *in situ* polymerization, inclusion encapsulation in cyclodextrins, freeze drying.

Nevertheless, the complexation of polyphenols with metal alkoxides by mechanochemistry has not been used as an alternative to enhance the properties of polyphenols until now.

In the present invention, a stable colored metal complex is produced without using solvents (or with a minimal amount of solvent as processing additive) by the reaction of polyphenols and/or β-diketones with metal alkoxides by mechanochemistry. The colored metal complex is stable under different conditions such as daylight, storage, and different formulations.

So far, there are no records or patents on using, e.g., lignin and curcumin, to produce colored materials with new value-added products that would circumvent the necessity of using petroleum-based chemicals for producing coloring matters (i.e., synthetic organic pigments or dyes including azo pigments are responsible for most of the organic red, orange and yellow pigments). On the contrary, all efforts are done to discolor or to bleach polyphenol compounds such as lignin or curcumin but not to produce other coloring matters from it. In the present invention, a polyphenol or a polyphenol and a β-diketone (e.g., lignin and curcumin from different natural and industrial sources) are used to react with metal alkoxides by mechanochemistry to produce homogeneous and stable colored metal complexes.

**Polyphenols as UV sun protection filter or absorber, antioxidant and skin protecting**

UV filters are compounds that absorb or reflect the UV rays from the sun or artificial light. They prevent the premature damage and aging of the skin and hair.

Several synthetic filters used in sunscreens generate allergic reactions, and often synthetic UV filters are prohibited or their maximum concentration allowed is adjusted. The consumers' apprehension of using synthetic UV filters and the urge to find natural products have redirected the attention to the use of natural UV filter or absorbers. Polyphenols have potential to be used as a UV filter such as curcumin, pongamol.

As an alternative, the EU cosmetic directive (v.1) lists some curcumin derivatives such as diacetylcurcumin and tetrahydrocurcumin diacetate, as UV absorbers.

EP0431755A1[13] discloses the sunscreen composition for topical application on human skin or hair comprising an effective amount of a substituted 1,3-diketone and a physiologically acceptable vehicle for the substituted 1,3-diketone. The substituted 1,3-diketone is curcumin and the physiologically acceptable vehicle is a solvent, a dispersant or a carrier to facilitate their topical use. Curcumin is used as a natural sunscreen filter. The process is clearly the physical mixture of two or more compounds without chemical bonding. In addition, metal alkoxide was not used in the manufacture as a solvent, additive, adjuvant or vehicle or emulsifier.

**As skin lightening**

Curcumin has been used in topical creams having a yellow color. Derivatives of curcumin such as tetrahydrocurcumin have been used for their skin lightening properties and aesthetic reasons due to this reduced yellowish color[14] (US5266344) or colorlessness unlike curcumin that is yellow colored.

**As a dye to be used as sindoor/kumkum or mehndi**

Although curcumin has a broader range of healthy properties, the use of red compounds, such as kumkum and sindoor (traditionally from turmeric using calcium hydroxides to have some instantaneous coloration), have been causing some skin diseases or allergies due mainly to the use of toxic red colorants such as azo or mercury or lead containing minerals that counteract the loss of redness of the material already degraded by alkanilization of the Curcuma.

Even henna intended to be applied for mehndi (temporary tattoo) may contain other ingredients such as *p*-phenylenediamine, pyrogallol and toxic textile dyes-- not permitted in cosmetics to be applied to the skin or even prohibited in cosmetics-- to improve durability or to produce different tones. Thus, a safe naturally based reddish- or brownish-colored material with reduced toxic potential would be of relevance for different cultural traditions.

**Keratinous dyeing**

One of the uses of the novel complex formed in the present invention is as a hair dye either as a powder or as a formulation containing the same. The method of applying hair dye is also disclosed. The metal-complex dye produced in the present invention and the uses as a hair dye and the hair dyeing processes here presented are unique, and there are no records or suggestions in the prior of art of the hair dye or dyeing techniques. The color itself is produced by the combination of two or three reactants or substances that confer to this dye the quality to be a good dye. The state of prior art on hair dyeing literature or patents related to the present invention only concerns the use of these three individual reactants, i.e. a β-diketone, a polyphenol, and a metal alkoxide, apart from each other in different hair dyeing systems.

Before Sir Perkin's discovery of the first synthetic dye in 1856, all dyes or pigments used were natural with or without mordants. After Sir Perkin's discovery, natural dyes were relegated due to their poor fastness to washing and to light. The hair dyeing and hair coloring industry is constantly growing, as is the awareness of the user and the professional personnel to use less risky and eco friendly formulations and methods of application[15].

Briefly, there are three basic categories of hair colorant: temporary, semipermanent and permanent, depending on the durability of the color in the hair after washing. If the classification is done depending of the mechanism involve in the production of the color, there are oxidative and non-oxidative hair dyeing methods. Temporal and semi-permanent hair dyeing are non-oxidative and permanent is oxidative hair dyeing.

A conventional permanent or oxidative hair dye system comprises three types of reactives: primary intermediates, color couplers and oxidizers. The primary intermediates are capable of undergoing oxidation and subsequent development of color such as o- and *p*-phenylenediamine, o- or *p*-aminophenol or *p*-toloenediamine. The couplers such as m-diamines, m-aminophenol and phenols, do not produce any color when oxidized alone, but form dyes when oxidatized in presence of a primary intermediates. Thus, the couplers produce hair nuances to imitate a naturally looking color when they react with the initial oxidation product to give highly colored indo-dyes.[16] Thus, they react with the base intermediates to produce a dye. Oxidizers are usually hydrogen peroxide.

The most permanent (oxidative) hair colorants use aromatic amine such as *p*-phenylenediamine (PPD) in order to achieve long-lasting hair color, and it is supposed to be the only way to successfully coloring grey hair. It is a suspected carcinogen alone or in combination with other components in the formulation, such as hydrogen peroxide produces other intermediates known to be mutagenic or carcinogenic.

The couplers, such as polyhydric phenols, i.e. resorcinol, pyrogallol, react with the intermediate products of the reaction with p-phenylenediamine to produce light brown colors.

US3506389 (GB1025916) discloses an oxidative hair dye with N,N-dialkyl-*p*-phenylenediamine used in conjunction with phenols, 1-3 diketones and/or pyrazolones and the color is developed by using hydrogen peroxide. According with Corbett,[16] this method form is essentially a application of the color photography process to the hair. Pyrazolones are always used in conjunction with β-diketones in oxidative hair dyes to produce azomethine dyes, which are analogous to those formed in the color photography process.

WO2006/106366A1 discloses an oxidative hair dye comprising a hair dye, an organometalic and an oxidating agent. The hair dye is a mixture of at least one developer and at least one coupler, such as those used in the conventional oxidative hair dye. The organometallic is a organo titanate such as tetraalkyltitanate (e.g. titanium alkoxide) and titanate chelates such as those commercially available and produced by Du Pont (preparation of many titanium chelates are reviewed in[17]: "Titanium compounds, Organic", Kirk-Ohmer Encyclopedia of chemical technology vol. 25).

Thus, developer and couplers are intentionally used to produce diverse colors in the final hair dye. Even the most preferred organometallic compound was aqueous diisopropyl di-triethanolamino titanate (Tyzor TE). Thus, a chelate that can release the triethanolamine upon hydrolysis and may lead to increase the pH as in the case of using ammonia. They describe that the organotitanate intensify the color of the initial permanent hair dye system (having developer and coupler). Thus, the color is not produced by the organometallic compound as mentioned in the discussion of table 1 of 66A1. The authors affirm that the organotitanate used without the presence of additional oxidative hair dye does not act as a dye, i.e. imparting colour to the hair.

In the present invention, the color is produced without the use of the conventional dye precursors (primary intermediates or couplers). It results from the reaction of a β-diketone and a metal alkoxide or the reaction of a polyphenol and a metal alkoxide. Hence, amino containing compounds and conventional oxidizer are non-essential for producing color.

In one system of the present invention, curcumin -a direct dye- and quercetin -a polyphenol- are used for producing color, by reaction with the metal alkoxide either in the hair or applied in the hair either after mixing the reactants or after the final powdered complex is obtained with a suitable functionalization of the surface, if desired. Only direct dyes are used instead of oxidation dyes (having primary intermediates and couplers). It would be less risky for the consumer and the professional to deal with a hair dye product and process that does not contain aromatic amine or alkanolamine or any amino-containing compound to produce the color rather than those commonly used in oxidative hair dyeing. Even healthier would be the use of a hair dye system without oxidizer that forms hazardous and mutagenic intermediates such as those produced in conventional hair dye constellations.

In view of the high toxicity of the conventional oxidative hair dyes, it would be better to return to ancient practices where direct dyes or natural dyes were used for coloring keratinous materials such as henna (lawsone), walnut tree or shells (juglone), chamomile (apigenin), logwood (hematin). Curcumin is less used as natural hair dye due to fugitive properties: it fades under air; it is unstable at different pH conditions, as explained before.

Natural dyes are also employed as combinations of several natural dyes and mordants such as salts of aluminium, chromium and iron and tannins. Metal salts are used for fixation of the dyestuff and increasing the fastness properties. In particular iron mordants, leads to color differences with darker shades. However, many of these salts precipitate in the hair dyeing formulations or are water-soluble and drip out the hair when weathering or sporting.

Conventional hair dyes, such as those using henna, are mixed with metal salts as mordants and have several drawbacks, such as loss of color due to water or shampooing. Henna is only adhered to the hair and not absorbed. Even peroxides and arylamines are added to the henna formulations to improve the dyeing behavior. A major challenge of natural dyeing is that only very few sources for red and blue are available.

Commonly, turmeric is used in combination with other natural dyes to produce several colors such as Carthamus tinctorius (safflower) that gives a scarlet, with indigo to produce olive green. These are beautiful as textile dyes, but they do not stand washing even after using salts as mordant. For hair dyeing, the process is even less suited due to the necessary mild and healthy conditions that the human keratinous dye requires. The extent of the applicability of natural dyes in the dyeing industry is limited predominantly due to the small range of colors and laborious and time-consuming methods of application.

The use of metal complexes as dyes owes its origin to the mordant dyeing. In regard to metal-complex dyes *"Ullmann's encyclopedia of industrial chemistry",* describes extensively their use of metal chelation of azo/azomethine dyes. The anionic 1-2 chromium complex with salicylic acid, sodium diaquabi[salicylato(2-)O1,O2] chromate(1-), is generally used as a precomplex to synthesize 1-2 chromium complexes of azo dyes in aqueous solution at high pH for introducing hydrophilic groups in the complex with outstanding wet- and light fastness. This is the so called chromium(III) salicylic acid method of chromatation. The complex formation on the salicylic acid group has little influence on shade and lightfastness contrary with the azo compounds.

As a result, many formulations of natural hair dyes are combined with oxidative hair dyes to achieve higher color intensity, stability and durability. Nearly all chromophores systems commonly used in synthetic dye chemistry (nitro, azo, anthraquinone, triphenylmethane, azomethine) are used. Thus, toxic colorants are generally masked with the name "natural," while containing all reactants commonly found in conventional oxidative hair dye.

Thus, colorants with enhanced properties that combine the benefits of a natural colorant with green chemistry synthesis with effective and healthier properties are needed not only in the dyeing process of different substrates but also in the industry. Moreover, the addition of a metal alkoxide never used for dyeing with the formation of a color that are difficult to obtain in the nature is disclosed in the present invention.

In the present invention, direct dyes such as curcumin are used for the formation of color with or without other polyphenols or other natural colorants. The reaction is produced either into or onto the hair by reacting with a metal alkoxide. This hair dyeing system is novel and, when applied to the hair by several methods, imparts a semi-permanent or temporary color.

## Catalyst for reactions such as polymerization

US5756610 disclosed a process for the synthesis of a titanium (III) catalyst for the syndiotactic polymerization of styrene without the cyclopentadiene moiety. The catalyst is formed by the reaction of an intermediate product and either aminosilane or alcohol in toluol. The intermediate compound is formed by the reaction of titanium trichloride with a β-diketone in tetrahydrofuran. The R group from the diketone was a C1 to C12 alkyl, aryl or alkylsilane.

The halogen compound was used for the source of titanium, which easily generated salts with other cations present in the reaction and had to be removed. The reaction is carried out in presence of a solvent which, generally speaking, has to be removed from the final product. Thus, the procedure is time-consuming and not ecofriendly.

The present invention comprising the synthesis of a metal complexes in powder form to be used as a catalyst for several reactions or polymerization is characterized by the one-step production of an ecofriendly catalyst for reactions of polymerization. Transition metal β-diketonates are already used to substitute the toxic mercury catalyst for the polyurethane formation such as titanium and copper.

In the present invention, a β-diketone and/or a polyphenol, e.g., the curcumin or lignin, are inexpensive and of low or no toxicity and is used to start the direct complexation with a metal alkoxide for further uses as a catalysts.

## Detailed description of the invention

The invention relates to a metal complex according to claim 1.

The invention further relates to a process according to claim 6. In particular, the present invention deals with the mechanochemical synthesis of metal complexes by using e.g. curcumin and/or quercetin and/or ferulic acid and/or lignin and/or ellagic acid and at least one metal alkoxide as starting materials.

## β-diketones and polyphenols

A β-diketone is a diketone in which the two keto groups are separated by a single carbon atom. In this view, a β-ketoester, a thio β-diketone and a β-ketoamine (Shiff base) behave similar to a β-diketone since they contain a reactive hydroxyl group which is prone to react readily with metal alkoxide.

The β-diketonate ligand has the ability to coordinate with most elements which is a feature that distinguishing it from other organic ligands.

β-diketones undergo keto-enol tautomerism. Tautomerism implies the presence of the ketone and the enol forms in equilibrium. Pentane-2,4-dione (more informally acetylacetone) is the β-diketone that has been most studied. The feature of β-diketonate chemistry, in particular of acetylacetone, is the tendency to form inert (at least for catalyst) tri or tetramer complexes with a metal for most transition and main group elements.

The β-diketone compound to prepare the metal β-diketonate complexes of the present invention may be selected from:

1. synthetic β-diketones either with modification of the methylene group or with the introduction of nitrogen or sulfur groups into the keto-enol moiety such as: pentane-2,4-dione (acetylacetone), 3-Y-pentane-2,4-dione (wherein Y = Cl, Br, Me), benzoylacetone, benzoylacetone, dibenzoylmethane, diisobutyrylmethane, 2,2-dimethylheptane-3,5-dione, 2,2,6-trimethylheptane-3,5-dione, dipivaloylmethane, trifluoreacetylacetone, hexafluoroacetylacetone, benzoyltrifluoroacetone, pivaloyltrifluoroacetone, heptafluorodimethyloctanedione, octafluoropentane-2,3-dione, 4H,4H-decafluoroheptan-3,5-dione, 2-thenoyltrifluoroacetone, 2-furoyltrifluoroacetone, 2-thenoylacetone, 2-furoylacetone, cycloheptane-1,3-dione, thio-β-diketones, chiral β-diketones, and mixtures thereof.

2. naturally occurring β-diketones such as:

(a) curcumin and its derivatives such as from curcumin species (Zingiberaceae): curcumin from turmeric rhizome; curcumin, demethoxycurcumin, bisdemethoxycurcumin or mixtures thereof; curcumin metabolites such as tetrahydrocurcumin, curcumin glucuronide; synthetized curcumin and mixtures thereof; other curcuminoids such as cyclocurcumin, cassumunin A, cassumunin B, cassumunin C.

(b) β-diketones derived from leaf wax of Festuca Ovina, from epicuticular waxes of barley or from epicuticular waxes of vanilla bean species.

(c) Pongamol from pangamia glabra fruits, (+)-usnic acid from Usnea dasypoga, clusianone and 7-*epi*-clusianone from Guttiferae (Clusiaceae)

(d) Any other compound able to form keto-enol tautomerism

Pettinari C.[18] et al. reviewed the syntheses of β-diketones and metal β-diketonates. β-diketones may also be obtained by *in situ* synthesis prior or during the complexation of the present invention.

Curcumin, a β-diketone with sterically bulky functional groups, can provide an alternative route, for instance, for catalysis chemistry by its complexation with metals. The diarylheptanoid moiety of curcumin comprises both aryl groups tethered by a linear seven carbon chain having substituents. Both β-diketone substituent and the diarylheptanoid with the double bonds make curcumin one of the most ubiquitous compounds for several uses such as pharmaceuticals, cosmetics, foods, coatings, catalyst, reagents for film deposition.

Since curcumin is both a β-diketone and a diarylheptanoid, other diarylheptanoids having β-diketone substituents to produce the metal β-diketonate complexes may be selected from naturally occurring diarylheptanoids such as those isolated from Zingiberaceae, Centrolobium Sclerophyllum, Betulaceae, Myricaceae, Juglandaceae, Aceraceae or synthetic diarylheptanoids.

Phenolic compounds or polyphenols comprise a wide variety of molecules that have several hydroxyl groups on aromatic rings such as lignins but also molecules with one phenol ring such as phenolic acid and phenolic alcohols.

The term polymeric phenols is preferred to polyphenols because otherwise lignin would be excluded according with the strict definition of polyphenols. Polyphenols in line with WBSSH definition exclude many compounds purely based on the solubility or molecular weight. Quideau et al[19] (2011) proposed the definition of polyphenol as plant secondary metabolites derived exclusively from the shikimate-derived phenylpropanoid and/or the polyketide pathway(s) featuring more than one phenolic ring and being devoid of any nitrogen-based functional group in the most basic structural expression. In a recently book edited by Watson R. R.,[20] Tsimogiannis D. and Oreopoulou V. in chapter 16 *Classification of phenolic compounds in plants* revised the classification of polyphenols according to Harborne.[21] Thus, the synthetic or naturally occurring polyphenol compound from plants or animals (e.g., insects and crustaceans) to prepare the metal polyphenol complexes of the present invention may be selected from:

Simple aglycone or glycone phenols e.g. arbutin;

Phenolic acids, e.g. vanillic acid and syringic acid;

Phenolic aldehydes, e.g. vanillin and syringaldehyde;

Phenylethanoids (phenylacetic acids, acetophenones, phenethyl alcohol derivatives), e.g. tyrosol;

Phenylpropanoids (hydroxycinnamic acids and derivatives, cinnamic aldehydes, monolignols, phenyl propenes, coumarins, isocoumarins, chromones), e.g. ferulic acid, *p*-coumaryl alcohol, cafeic acid and rosmarinic acid;

Stilbenoids, e.g. resveratrol;

Anthraquinones and anthrones, e.g. aloe emodin, alizarin, purpurin, carminic acid;

Xanthonoids, e.g. mangiferin;

Naphthoquinones, e,g, lawsone, alkannin and juglone;

Raspberry ketone;

Open bridge flavonoids (chalcones, dihydrochalcones, and the keto-enol tautomers β-hydroxychalcones and β-ketodihydrochalcones), e.g. 1-(2-hydroxyphenyl)-3-phenyl-1,3-propanedione isolated from the root bark of *Pongamia pinnata* or the stem bark of *Millettia ovalifolia;*

Closed bridge flavonoids (aurones, auronols, flavans, flavan-3-ols, flavans-4-ols, flavan-3,4-diols, flavones, flavonols, flavanones, anthocyanidins, flavanonols, isoflavones and neoflavonoids), e.g. cathechin, epicatechin, epigallocatechin gallate, quercetin, chrysin, rutin, naringin, hesperidin;

Diarylheptanoids, e.g. curcumin;

Phenolic dimers and condensation oligomeric products such as ellagic acid, lignans, biscoumarins, dimers and trimers of ferulic acid, cafeic acid oligomers, bis-xanthones and xantholignoids, dimeric or oligomeric stilbenoids, bisantraquinones, dianthrones, di and oligomeric stilbenes;

Polymeric phenols (tannins and lignins), e.g. gallotannins, elligatannins, proanthocyanidines, phlorotannins, thearubigins, kraft lignin, lignosulfonates;

Hybrid phenolic (phenolic terpenes and phenolic lipids), e.g. carvacrol, thymol, THC, CBG, carnosic acid, carnosol, cardanol, bilobol, gingerols, shogaols, ginkgolic acids;

Aqueous or organic extracts or tinctures or powders of polyphenols are commonly composed of a mixture of several polyphenols as occurs in nature, e.g. extracts or powders from plants of genera: *Curcuma, Lawsonia, Indigofera, Genipa, Oenothera, Lespedeza, Passiflora, Hamamelis, Theobroma, Coffea, Chamaemelum, Quercus, Zingiber, Capsicum, Malva, Bixa, Tagetes, Cynara, Glycine, Asperula, Angelica, Hieracium, Ammi, melilotus, Aesculus, Lithospermum, Solidago, Origanum, Camellia, Schisandra, Hibiscus, Rosa, Ribes, Acacia, Bactris, Rhus, Gingko, Juglans, Hibiscus.*

The chemical composition of polyphenols in nature commonly is a mixture of several polyphenols or is conjugated with sugar and organic acids.

Some of these polyphenols such as lignin may be selected from the milled wood lignin (from milling wood or chips), or the by-product of the treatment of the lignocellulose material in the paper industry. Chemical agents such as caustic alkalis, ammonia, chlorite, sulfur dioxide, diluted and concentrated acids and solvents are commonly used to produce pulp and paper. These agents commonly modify the lignin and should be eliminated from the end product, but they are not entirely eliminated and then reduce the application potential of lignin. Ligninsulfonates are obtained from the (acidic) sulfite process; kraft lignins are the product of sulfate pulping process; soda lignins are sulfur free; and organosolv lignins are extracted by solvents and are of high purity.

Therefore, in the present invention, the term polyphenols refers also to extracts of materials containing polyphenols which have been subjected to the complexation procedure of the present invention. In addition, the polyphenol compounds of the present invention include polyphenols that are subjected, prior to the complexation of the present invention, to another kind of chemical, biochemical, enzymatic, or physical procedure or complexation. As further presented in this invention, the encapsulation of polyphenols is a strategy to enhance the properties of polyphenols. Thus, polyphenols which are modified or protected are also used as a source of polyphenol in the present invention. Similarly, β-diketones which are protected (e.g. as enol ethers or enamines) or manipulated may also be used as a source of β-diketones compound in the present invention.

Lignin may be selected from a native lignin, an enzymatic lignin, an alkaline lignin, a lignosulfonate (as a sodium-, calcium-, magnesium-, or ammonium salts) or a kraft lignin or an organosolv lignin.

Polyphenols have several health benefits for humans and animals due to several properties, including antioxidant, anti-inflammatory, cardioprotective and neuroprotective functions. Besides, polyphenols are antibacterial, anti-viral and anti-fungal. These capabilities of polyphenols are key to their use for treatment of several diseases, in food-processing and for anti-aging purposes in various cosmetic and pharmaceutical formulations. However, polyphenols are sensitive to several environmental factors such as light and heat and may degrade rapidly under water, air or storage conditions.

The present invention provides a new synthetic approach to stabilize polyphenols and/or β-diketones by complexation with metal alkoxides with an excellent stability e.g. to environmental and storage conditions. These novel metal complexes can be further encapsulated, e.g., by miniemulsion polymerization for further applications.

**Metal Alkoxides**

According to IUPAC,[22] the term alcoholates is synonymous of alkoxides. Alcoholates should not be used for solvates derivate from an alcohol such as $CaCl_2.nH_2O$, for the ending -ate often occurs in names for anions. Metal alkoxides[23] or metal alcoholates have the formula $M(OR)_x$ where $M$ is the metal (or non-metal or other cationic species), $R$ is an organic radical and $x$ corresponds to the valency of the metal $M$, the variables R, x and M are as defined in claim 1. The metal alkoxide can be either in solid or liquid form. Disclosed herein is that they are produced from almost any metal of the periodic table of the elements. According to the invention defined in the claims, M is titanium or one M of a heterometallic alkoxide is titanium. Both metal and radical confer properties of the alkoxide. The metal provides the electronegativity, whereas the radical provides the acidity and the ramification. Thus, metal alkoxides are so versatile and diverse that they can be either water-soluble or water-sensitive. One of the advantages of alkoxides is that they only form their parental alcohol as a by-product. The health hazard of metal alkoxides depend on the metal they contain and the alcohol they produce after hydrolysis. The chelates with which the metal alkoxides are formed, e.g., β-diketones, β-ketoesters are generally more stable than the alkoxides themselves.

$M(OR)_x$ or $[M(OR_x)]_n$ is a metal alkoxide or heterometallic alkoxide such as mixed alkoxide mixed halide-alkoxides or bimetallic alkoxide (double alkoxide), or polymeric metal alkoxide or oxo metal alkoxides metal aryloxide or bi-, tri-, and tetrametallic alkoxides or adducts with neutral ligands where

(a) according to the claimed invention, M is titanium, or one M of the heterometallic alkoxide is titanium. Disclosed herein is also that M is one or more elements from the elemental periodic table, preferably zirconium, hafnium, vanadium, aluminium, germanium, silicon, niobium, lithium, tantalum, zinc, magnesium, antimony, indium, gallium, copper, holmium, tin, lanthanum, erbium, barium, gadolinium, yttrium, tantalum, dysprosium, cobalt, tellurium, lead, bismuth, calcium, cerium, iron, strontium, molybdenum, tungsten, neodymium, nickel, samarium, europium, osmium praseodymium, boron, sodium, potassium, thallium, copper, scandium, nickel, chromium, manganese or mixtures thereof;

(b) R is an organic radical such as methoxide, ethoxide, propoxides (n- and iso-), butoxides (n-, iso-, sec-, and tert-), amyloxides (n-, sec-, tert-) and neopentyloxides, aryloxides;

(c) $x$ corresponds to the valency of the metal M and

(d) n corresponds to the degree of molecular association

The term heterometal alkoxide with bi-, tri-, and tetrametallic alkoxides (as explained in the corresponding chapter in *Alkoxo and Ariloxo Derivatives of Metals* by Bradley D. C. et al) such as bimetallic alkoxides $M_n M'_m (OR)_p$ is adopted here since their structure and physicochemical properties belong to the same types as those of homometallic ones. A metal alkoxide or a heterometal alkoxide is not a salt since they do not proceed from the reaction of an acid and a base neutralizing each other.

Polymeric metal alkoxides $[M(OR)_x]_n$ are the product of partial hydrolysis or thermolysis of $M(OR)_x$ where n is corresponds to the degree of molecular association.

Adducts of metal alkoxides with ligands such as $M(OR)_x \cdot mL$ where m is the composition of the solvates. Optionally, the metal alkoxide used in the present invention can be prepared *in situ* by reaction of alcohols with metals, with metal hydroxides, with metal halides, with metal amides or metal alkoxide of other alcohols or mixed halides alkoxides or by alcoholysis and transesterification reaction and others methods (alkoxides, metal in Kirk-Othmer Encyclopedia of Chemical Technology, 4th ed. vol 2).

The metal β-diketonate complex synthetized in the present invention is the product of the oxygen-bonded β-diketonate complexation between a β-diketone and a metal alkoxide promoted by mechanochemical synthesis to assure homogeneity of the final product

The metal polyphenol complex synthetized in the presence invention is the product of the reaction of a polyphenol with a metal alkoxide.

Both β-diketonate and polyphenol may be mixed before the reaction with the metal alkoxide. In this way, even more different complexes and colors are obtained.

In the present invention, the mechanical grinding is preferably achieved by a simple hand mortar and pestle. Various mechanical deformation methods, well known in the prior art, can be used such as mechanical mortar and pestle, high speed milling, ball milling, attrition milling and planetary milling.

The process of the invention may comprise the following steps:

1. At least one metal alkoxide is added to the polyphenol or added to the β-diketone and polyphenol (or vice versa) in the desired stoichiometric ratio metal alkoxide to β-diketone and/or metal alkoxide to polyphenol, such as between 1/100 to 100/1 or vice versa. Both reactants are homogeneously mixed before the following step 2. In some cases where the molecular weight is not exact known, e.g in the case of lignin, weight ratio metal alkoxide to β-diketone and/or polyphenol between 1/100 to 100/1 may be used.

a. Optionally, depending of the further use of the metal β-diketone or polyphenol complex as a sunscreen, dye or colloids, an amount of additive between 0,01 wt.% and 50 wt.% of the total weight of both reactants is added. The additive compound is selected from the following either as a single compound or a combination of two or more compounds selected from the group of further β-diketones, further polyphenols, further metal β-diketones, further metal polyphenols, solvents, fatty oils, fatty alcohols, organic acids, vitamins, aminoacids, lipids, proteins, carboxylic acids, synthetic or natural colorants, wetting agents, swelling agents, penetrants, pH regulators, surfactants, perfumes, thickeners, milling adjuvants, salts, oxides, water.

b. Optionally, the additive is added to the reactants taking account of the compatibilities.

c. Optionally the additive is added simultaneously with the reactants.

2. The abovementioned mixture may be ground by hand or mechanically, so that the mechanochemical reaction occurs between the two reactants. Several mechanochemistry methods can be used, such as hand and mechanical mortar and pestle, ball milling, attrition milling, planetary milling. The mechanochemical reaction gives a colored homogeneously and finely dispersed powder material. The color in the final powder depends of the metal used and the radical of the metal alkoxide as well as the β-diketonate and the polyphenolic ligand.

3. Optionally the additive is added after the complex is formed.

4. The abovementioned mixture may be let to rest (such as for 1 hour).

5. The resulting, finely dispersed and colored powder is ready to be used in food, cosmetics, pharmaceuticals, paints, and other applications.

The β-diketones and/or the polyphenols and the metal alkoxide act as reactive compounds prone to react in a well-known synthetic route. The mechanochemical synthesis enhances and promotes the reaction of both reactive compounds and provides the desired colored product or product precursor for further uses such as sunscreens. All reactive compounds used for the process of the present invention can be either in solid or liquid forms under the conditions used for the mechanochemical synthesis. In addition, one or more reactive compounds for the process of the present invention can be used in gaseous state under the conditions used in the mechanochemical synthesis, as long as at least one other compound is either solid or liquid under the conditions used in the mechanochemical process.

The mechanochemical route of synthesis is a direct synthesis that uses either manual or mechanical milling or grinding to initiate or facilitate the process in solid state or solid-liquid state. Either solid-solid synthesis without solvent or with minimal amounts of solvent or solid-liquid synthesis is used to promote mechanochemical reactions. The most important advantage of mechanochemistry is the solvent-free synthesis or near solvent-free synthesis with only small amounts of solvents (i.e. less than 10 wt% based on the total of weight of the reaction mixture), the so-called liquid-assisted grinding. According to Tanaka,[24] the solvent-free term refers to the stoichiometric application of solid or liquid reagents with less than a 10% excess of a liquid or soluble reagent and/or less than 10% of a liquid or soluble catalyst. Minimal amount of solvent implies that no solvent is a priori and deliberately added to the reaction that could require solvent-consuming purification steps after the reaction.

Solvent-free organic synthesis is eco-friendly and obviates the necessity of further steps of solvent evaporation and recycling of the solvent. In addition, the amount of hazardous by-products that can interact with the solvent is decreased.

In contrast to mechanochemistry, the traditional production of metal β-diketonate complexes by solution-based methods uses substantial amounts of solvents, generally organic such as toluene or benzene in order to remove the alcohol produced during the reaction and to complete the reaction. Consequently, the solvent has to be removed by different methods such as distillation, distillation under reduced pressure or vacuum extraction. This process is tedious and involves careful handling of toxic compounds. In the present invention, since both the metal alkoxide and the β-diketone and polyphenols are sensitive to solvents, it is beneficial to let react only by mechanochemistry.

Mechanochemistry can promote reactions quickly and in large quantities. The effectiveness of the mechanochemical reactions depends on the chemical and the mechanical properties of the agents or reactants. Mechanochemical phenomena may lead to the activation of strong covalent chemical bonds by the presence of an external mechanical force. However, more labile non-covalent bonds may be also activated e.g. supramolecular materials.

Although the theory of the mechanochemical reactions is still in its infancy and, so far, there is not a general theory for mechanochemical reactions, some possible phenomena may involve:

formation of active surface radicals; modification of physicochemical properties; enhancement of reactivity due to stable changes in the structure; enhancement of effectivity in the solid phase than in the liquid phase.[24, 25]

The mechanochemical reactions of the process of the present invention can be made by various methods.

Prior to starting the mechanochemical process, a detailed control of the form of the reactive compound should be effected. If both reactive compounds are grove solids, they can be ground separately before the mechanochemical process begins. Optionally, if possible, a slight increase of the temperature for allowing to produce the homogeneous mixture would be convenient. The starting materials are combined at slow speed and then ground together at higher speed. The grinding can be done under dry or wet conditions. A dry grinding is preferred. However, in case wet grinding is required, inert grinding aids that do not react with either the reactant compound or the final product are preferred.

The energy of the grinding process can be varied within wide ranges. Low energy ball-milling, attrition milling, vibratory milling and similar low energy grinding processes known in the art of grinding are preferred over high energy milling processes, since the use of a grinding medium at high energy can produce wear and, thereby, contaminate the reactant and the product obtained. However, when high energy milling is needed, high energy ball milling, high energy planetary milling and similar can be used. Grinding media that do not react with the reactant compound and product are preferred and can be agate or similar materials. To increase the reactivity of the reactant compounds or to induce melting of one or more reactants if need be, the milling process can be carried out at high temperatures or the reactive compounds can be pre-heated prior of the start of the milling process.

Additives such as cutting, milling and grinding aids, lubricants, surfactants, polymers and antistatic agents may be used to prevent agglomeration of the particles and, thus, improve the grinding efficiency. These additives can be in the form of a liquid, a solid, a semisolid, a waxy substance, flakes and micronized beads. A great variety of substances can be used to enhance the milling process. A careful selection with regard to the impact on the quality of the final product as well as environmental issues must be considered. These additives may be selected from the group consisting of ethoxylated alkyl phenol (such as Dodoxynol-5, 6, 7, 9, 12 and Nonoxynol-9, 30), fatty alcohol ethoxylated (such as emulan OG or emulan TO 40), sodium dodecylbenzenesulphonate, sodium dodecyl sulphate, maltodextrin, lecithin (such as phosphatidylcholine, hydroxylated lecithines), fatty acids (such as stearic acid, oleic acid), polysorbates and similar. These additives may be used in a concentration between 0.01 and 10 wt% based on the total weight of the reactive mixture. Depending on the reactivity of the milled materials and the intensity of the milling process, the milling time can vary between 1 minute and 1 hour.

Mechanical grinding device which controls parameters such as mortar and pestle speed is more appropriate for the synthesis due to the ease of standardization and reproduction of results. For small quantities or at laboratory scale, a micro mill where the speed of both mortar and pestle can be controlled is preferred. In addition, the mortar and pestle could be covered with a transparent cover for safety. A simple hand mortar and pestle, preferably from agate to avoid contamination, is sufficient for small quantities of sample.

The process of the present invention enables the preparation of metal polyphenol complexes and metal β-diketonate

polyphenol complexes with high yields and under ecofriendly conditions as well as the immediate synthesis of a ready sample for further analysis such as powder X-ray diffraction (XRD) studies. The process of the present invention obviates the troublesome processes to purify the product in solution-based methods or in mechanochemical methods using large amounts of additives. The metal complex in powder form is ready to be characterized by several analytical techniques such as XRD or spectrophotometric measurements.

For XRD studies of the metal complexes of the present invention, a drying process may optionally be carried out at the boiling point of the parental alcohol or at reduced pressure, among others. In this way, the water or parental alcohol, that might be produced in the reaction, is eliminated. However, the direct characterization of the metal complex after the mechanochemical reaction without removal of the volatile by-products or without dissolution or recrystallization in other solvents is preferred.

In fact, the product of the present invention, the metal β-diketonate/polyphenol complex in powder form, can be crystalline, semicrystalline or amorphous depending on the selection of the reactants and the molar ratio of the reactants used. If two different metal β-diketonate/polyphenol complexes--with the same or different metal atom-- obtained separately by mechanochemistry and by redistribution reaction, are allowed to react by mechanochemistry, cocrystalline complexes can be obtained.

For further applications of the metal β-diketonate/polyphenol complex produced in this invention, such as precursors for film formation techniques or metal oxides with a treated surface such as those to be used in sunscreen or ceramic production, partial or total elimination of the organic material can be conducted. Since the final metal β-diketonates/polyphenol complexes contain mainly carbon, oxygen and hydrogen and the metal or mixture of metals (such as those from curcumin and metal alkoxide), the calcination or pyrolysis of the organic material proceed straightforward. The temperature used for the decomposition of the product prepared by the mechanochemistry method is preferably above 300°C for three hours, more preferably about 600°C for three hours, and most preferably about 900°C for 3 hours. Depending on the film deposition technique to be used, subsequent thermal treatments and dwell times can be optionally adjusted. Thus, if more crystallinity is desired or required, the final metal β-diketonate/polyphenol complexes can be sintered.

## Mechanochemical reaction of titanium alkoxide with curcumin

Mechanochemical synthesis of the sole reactants titanium n-butoxide and curcumin in a molar ratio titanium n-butoxide to curcumin of 1:1 in a mechanical agate mortar and pestle led at the beginning to an orange reddish paste which turned red after three minutes of constant milling. The orange reddish paste was easily milled to a fine red-colored titanium-curcumin complex. The complexation does not need any additional catalyst. Titanium n-butoxide acts as a catalyst and as a metal precursor. No separation of phases occurred. The titanium-curcumin complex produced in the present invention is red-colored, whereby the red coloration is not due to external influences such as acids, bases, solvents or catalysts. Only the two reactants -curcumin and a metal alkoxide- are the starting materials.

## Use of the metal complexes for sunscreens with concealer effect

Many people are reluctant to use sunscreens that leave a whitish color or chalky look on the skin. Most natural sunscreens in the market have this disadvantage. The problem is compounded if high amounts of pigments or dyes are added to the sunscreen (to produce the so-called foundation) since it often generates aggregation or irregularities in the sunscreen itself or in the skin and the masking effect is lost. Thus, the desired final naturally looking texture of the sunscreen is not achieved.

Curcuminoids have been used as skin protectant in cosmetic formulations. For instance, Vicco Turmeric Cream used curcumin or turmeric in formulations. These formulations are yellow colored and perhaps undesirable in cosmetic preparations because yellowing is usually associated with spoilage. Efforts have been made to change the color of curcumin by synthetic treatment. Thus, as mentioned previously, tetrahydrocurcumin or tetrahydrocurcuminoids has been used also as skin lightening taking advantage of its colorlessness or off-white color.

As previously explained, curcumin changes color with the change of the pH. However, this change is not stable and curcumin degradation begins quickly.

Traditionally in many countries, curcumin is mixed with a base, such as calcium hydroxide, to produce a red colored paste to be used as a colorant for bindi. However, this mixture is very unstable as it is only a product of acidity or basicity of the medium or the physical impregnation and people add different red colored matter such as toxic colorants in order to produce red color.

There is no evidence of red colored curcuminoid in the market by chemical or biochemical procedures with outstanding stability in different media and that could be used with less risks around the world.

One of the objects of the present invention is to use the metal β-diketonate/polyphenol complexes, in particular metal curcumin and/or ferulic acid and/or quercetin and/or ellagic acid and/or lignin complexes, having metals such as titanium, zinc, cerium, iron, aluminium, zirconium, silicon, or germanium and mixtures thereof as a sunscreen. The use of this product as a sunscreen is characterized by beautiful purple or red coloration (or their shades) that is distributed homogeneously on the skin. In addition, the use of the metal β-diketonate/polyphenol complexes of the present invention as a sunscreen shows excellent stability in the vehicle without forming precipitates or aggregates in an homogeneous composition. With little changes in the composition of the reactants of the complexes and in the conditions of the reaction and the vehicle of the formulation used, a wide range of skin tones are achieved in line with guides used in the cosmetics to match the color of the skin.

The product of this invention to be used as a sunscreen is further formed by the mechanochemical reaction of β-diketone and/or polyphenol (e.g., curcumin, quercetin, ferulic acid, ellagic acid, and lignin) and metal alkoxide. Optionally, between 0.01 and 50wt% of the mixture of the β-diketone and/or polyphenol and metal alkoxide of an additive such as perfume (e.g. pentane-2,3-dione), solvent (e.g. pentane-2,4-dione), acids (e.g. ascorbic acid, tartaric acid, citric acid, acetic acid), fatty alcohol (e.g. cetyl alcohol), natural oils or extracts (e.g. plukenetia volubilis seed ) can be added to the prior mixture to impart different colors to the final product. The additive or mixtures of additives can be added conveniently to the metal alkoxide or to the β-diketonate and/or the polyphenol. Additives may be preferentially inert but it is not restrictive. A controlled reaction with the additives may be advantageous to generate other properties, if desired.

The metal β-diketone/polyphenol complexes having the additive are then mixed with a vehicle or carrier to produce a sunscreen, a foundation, or a hair dye. The vehicle or carrier can be any appropriate material such as any base cream, oil, gel or powder.

The vehicle substance or base can be selected from:

- a cream base for preparing skin or hair creams without active ingredients, without perfumes, without parabens such as cetomacrogol crème commercially available, comprising water, decyl oleate, cetaryl alcohol, cetearth-20, sorbitol, sorbic acid, or any cetomacrogol crème;
- a pasty fatty substance such as waxes, gum or mixtures thereof; an oil, a fatty alcohol, a surfactant, a gel, a powder, a UV filter and/or UV absorber, a skin or hair protecting, an emollient, an humectant, an emulsifier, a skin or hair conditioning, a refatting agent, a masking agent, an emulsion stabilizer, a cleansing agent, an antioxidant, an opacifying agent, a solvent, a viscosity controller, a bulking agent, an abrasive, an anticaking agent, a preservative, a parfum, a buffer agent, an antimicrobial, a salt, water or mixtures thereof or any other cosmetically or pharmaceutically acceptable vehicle;

In addition, the desired sun protection factor can be optionally adjusted by the addition of further synthetic or natural filters.

The weight ratio of the metal complex to the vehicle can be between 1:100 to 100:1. Preferably, weight percentage of the metal complex is a range between 0.01 to 30 wt% depending of the solar protection factor that is desired.

The active ingredient for the sunscreen is the titanium complex, or zinc-titanium complex or mixtures thereof. Optionally, another metal or mixture of metals can be used.

If niobium n-butoxide instead of titanium n-butoxide, and curcumin is used, an almost instantaneous red paste that solidifies into a dark red bordering black powder is achieved (not according to the claimed invention).

This invention covers a mechanochemical process for the synthesis of metal complexes from a polyphenol or from a β-diketone and polyphenol and a metal alkoxide. The present invention of metal complexes of polyphenol or of β-diketone and polyphenol is characterized by a solvent-free synthesis or near solvent-free synthesis with only small amounts of additives. This solvent-free organic synthesis is ecofriendly and avoids the necessity of further steps of solvent evaporation and recycling of the solvent. In addition, the amounts of hazardous by-products diminish.

Thus, the entire process for the production of metal complexes of polyphenols or of metal complexes of β-diketones and polyphenols in powder form of my present invention fulfills the conditions to be considered green chemistry.

The simplicity, high yield, low cost and ease of scale up makes the process of the present invention to produce metal β-diketonate complexes and metal polyphenol complexes and their use as sunscreen with concealer very attractive for industrial applications.

A major advantage of the process of the present invention to produce metal complexes by mechanochemistry over conventional methods to produce organic-inorganic hybrid materials is the rapid completion of the reaction within a few minutes of grinding, the low polydispersity of the finely ground powder and the high stability and homogeneity of the complex obtained. In addition, the process of the present invention provides novel nanostructures similar to those obtained by solution-state methods for instance after pyrolysis. Since the use of solvent in the present invention is reduced to a minimum or the process is entirely solvent-free, no additional steps are necessary to remove it. In this way, the high yield process of the present invention to produce metal β-diketonate complexes and metal polyphenol complexes, in particular, metal curcumin complexes and metal quercetin complexes and metal ferulic acid complexes, metal ellagic acid and metal lignin complexes is cost effective.

Other advantage of the present invention is the produccion of metal complexes with a pleasant odor that does not resemble the original odor of the reactants,

## Semipermanent and temporary hair dyes

The urge of solving problems regarding use of less amount of primary intermediates and couplers without detriment to the performance of hair dye with healthier properties such as protecting the hair from the sun and balancing durability and safety of the dye should be the goal of hair dyeing production.

Natural colorants with synthetic procedures to boost in the lab the already known properties of natural constituents or to find new products or processes thereof has been my goal in the present aspect of my invention on hair dyes and other cosmetic products.

With light deviations of the formula, the composition of the present invention can be oxidative or non-oxidative. The total or partial diminution of potential allergens or carcinogens such as those containing amine, e.g. *p*-phenylenediamine, is achieved without detriment to the desired final color.

Hair dying compositions containing these new complexes and methods of application are also disclosed: Cosmetic dyeing compositions containing, in an appropriate medium and by functionalization of the surface, a complex formed by the reaction of a β-diketone and/or a polyphenol with a metal alkoxide.

The dying formulation of the present invention containing β-diketones and/or polyphenols and a metal alkoxide as active ingredient forms a stable colour without the use of amines or sulfur containing compounds or salts or oxides commonly used in oxidative hair dyeing. However, the use of these compounds is not restricted and they can be added, if desired, to add additional properties to the final product.

The dyeing product and dyeing process disclosed in the present invention covers all types of hair such as brown, blond, black, gray or damaged hair. The process and product of the present invention covers in particular curly and gray hair well-known to be very challenging to be colored. Curly and gray hair is covered with an environmentally friendly product and process yielding a durable and stable colored product.

The cosmetic dyeing composition of the present invention provides a strong wash fastness, and water repellency that makes the hair dye stable to weather or sporting such as surfing, swimming with resistance to washing out in salty or chlorinated water without detriment to the stability or colored dripping.

The process of the present invention can be used for all kind of keratinous materials such as eyelashes, eyebrows, skin, and nails.

In the case of curcumin and/or quercetin and metal alkoxide, the color is only formed by these two/three reactants. There are no amine-containing compounds, no-sulfur containing compounds, no salts, no mordants present in the present dye composition to produce color. However, these non-essential compounds may be used if desired. The composition of dyeing hair further consists of at least one coupler and at least one developer conventionally used in oxidative hair dying. Optionally, a coupler or a developer is appropriately mixed either with the β-diketone, the polyphenol or the metal alkoxide. Hence, an oxidating agent may be also used. Moreover, combining at least one basing agent with at least one oxidating agent, as in the standard practice, may be used, if desired.

## Process for dying in one step

One aspect of the present invention is a process of dyeing keratinous material by one step for instance by applying to the keratinous materials one of more cosmetic formulations containing the prepared product. Several products of the present invention may be applied taken together or separately.

Additives can be added before, during or at the end of the hair dyeing.

At least one complex formed in the present invention can be applied directly to the hair in wet (after the reactants are mixed and before the final powder is formed) or dried form (powdered form after mechanosynthesis with or without additives) or by using a suitable solvent once the complex is formed with or without surface functionalization. Optionally, the product of the present invention may be functionalized before applied to the hair in several forms such as paste, cream or colloids or the like as disclosed for the sunscreen formulation. Optionally the product of the present invention can be applied using an appropriate functionalization of the surface and a suitable solvent.

The present invention also provides a dyeing composition containing the hair dye comprising the complex produced by the reaction of at least one polyphenol or by the reaction of at least one β-diketone and at least one polyphenol with at least one metal alkoxide, at least on additive selected from the group consisting of a wetting agent, a swelling agent, a penetrant, a pH regulator, a surfactant, a perfume, a synthetic or natural colorant, a thickener, water, etc.

**Process for dyeing in more than one steps, in loco complexation hair dyeing (ILCHD)**

During the process call hereinafter *in loco* complexation hair dyeing, ILCHD, a β diketone and/or a polyphenol is applied or put in contact with the substrate such as keratinous material to allow the penetration in the cortex of the hair. Once in the fibre, a metal alkoxide is added to the substrate, a complexation is clearly formed and proven by the formation of color. Presumably, a small increase of the temperature -since the process is exothermic, at least for the complexation of β-diketone with metal alkoxide- enhances the diffusion into the hair cortex and the complex is formed *in loco.* Thus, the β-diketone and the polyphenol are derivatised or complexed *in loco* and it is confined into the hair. Once the complex is entrapped in the hair, it is not removed unless rinsing with shampoo.

The β-diketone and/or polyphenol is diffused into the hair by the addition of a solvent in sufficient amount to solubilize them (preferably warm water or warm alcohol). After the solvent is evaporated, the metal alkoxide is added. Some solvents or additives are allowed to stay in the keratinous material but preferably in a minimal amount.

Since the complexes formed are UV-VIS absorbing materials may be detected instantaneously. However, some gentle massage with the hands or comb, to stimulate a triboeffect as in mechanosynthesis, can be advantageous.

ILCHD process is advantageous in comparison with conventional hair dyeing:

No amine-containing compound or amino-phenol compound is needed to form color; However, their use is not restricted to ILCHD;
The formation of color or change of color is instantaneous. Less time consuming process or shorter leave-on times than in natural or oxidative hair dyeing;
Minimal or no damage to the hair;
Several surprising properties to the hair such as manageability of curly hair and relaxing of the hair.

Combination of different colors and shades combination can be formed only by subtle changes in the radicals of the β-diketones, the polyphenol, and the metal alkoxide used.

Some additives can be added to the reactants to generate diverse colors, shades, textures.

A semipermanent hair dye is achieved that does not wash out with water and lasts for more than six washings with or without shampoo. However to be more permanent hair dye, conventional hair dye procedures as known since the beginning of the 20th century can be applied.

Due to the use of metals such as titanium or zinc polyphenols or β-diketones and polyphenols the hair is protected against radiation such as ultraviolet. Additionally more benefits are added to increase the health of the keratinous material coming from the ingredients themselves or by combination effects such as antioxidant, antimicrobials, anti-dandruff, anti-acne, non-comedogenic properties.

The process of application of the hair dye is gentle and pleasing and can be done easily.

Gray hair is dyed to red, orange, brown and other color and shades.

The ILCHD is carried out at room temperature. However, the temperature can be varied to increase or decrease the rate of diffusion of the reactants or the complexes formed.

Optionally, as in oxidative hair dye, a pH modifier and an oxidizer can be used to increase or decrease the rate of diffusion of the reactants or the complexes formed.

For the color to develop, there is no reason to include amine-containing compounds in the formulation. However, they may be added in the β-diketonate compound or in the alkyl or aryl moieties of the metal alkoxides. Moreover, conventional base intermediates and couplers can be used as conventional hair dyeing strategies, and even the product of the present invention can be blended with a conventional hair dye or hair dyeing process to produce a permanent dye.

**Process of dyeing keratinous material with curcumin and quercetin and the metal alkoxide**

One aspect of the present invention is a process of dyeing keratinous material by one or more steps, by applying to the keratinous materials a curcuminoid such as curcumin extract freshly prepared or commercially obtained and/or a polyphenol such as ferulic acid, quercetin, ellagic acid, and lignin.

The turmeric fresh or boiled roots are triturated and passed by a sieve. The curcumin extract is then used for further ILCHD. In addition, other β-diketone/polyphenol extract, tincture or powders can be used for ILCHD. The hair may be or may not be washed beforehand. The curcuminoid and/or polyphenol extract is applied extensively in the hair and let to dry. Preferably the excess of curcumin and/or polyphenol that is adhered to the hair by no absorbed can be rinsed with water, if desired. The same procedure can be repeated several times, preferably twice. Thus, the hair has not only absorbed but adsorbed curcumin and/or polyphenol. Speculatively, the β-diketonate moieties may react with the disulfide bonds of the hair and absorbed into the hair shaft. Similarly, phenol moieties may react with the amine or sulfur groups of the aminoacids of the hair. A metal alkoxide, e. g. titanium ethoxide, is then applied as either as delivered or in combination with an additive added in the hair by using e.g. a plastic pipette or a dropper bottle or a bottle with orifice reducing dropper. Preferably the alkoxide should not be diluted because the solvent used may also solubilize the curcumin and bleed from the hair. The hair turns immediately red or violet after the application. After carefully massaging with the hands, simulating a pestle, the hair is pleasingly dyed. A leave-in time is set between 0.5 and 60 min, preferably between 5 and 30 min and more preferably 10-15 min. As some free alkoxide may be found, optionally more curcumin or polyphenol can be added to the hair. Finally, the hair is washed with or without shampoo and let to dry.

In this method of application of the hair dye system, there is not addition of alkali; no acid is used for developing color. Even more, not oxidating agent is used. However, it is not restrictive. Thus, the hair dye composition can further contain an oxidative dye, an alkaline agent and an oxidizer.

Because curcuminoids, i.e. polyphenols stands up well to heat processing, the temperature may be lightly increased to form different colors. However, the process is preferably performed at room temperature.

This is a semipermanent hair dye because it is a product that lasts for at least six washings with or without shampoo and does not use hydrogen peroxide to develop the hair color.

The compound produced in the present invention is a novel substance, firstly synthesize by the inventor with several advantages and with several fields of application.

In the present invention, the color in the complex is determined by the initial compounds, a β-diketone and/or polyphenol and metal alkoxide. A great variety of colors are produced without the use of primary intermediaries or couplers such as amine derivatives, peroxides or ammonia. Thus, the hair dye containing a least one β-diketone and at least one polyphenol may be safer and healthier for the user. Since the amount of ingredients present in the present invention is small, the reproducibility of the final color is higher and the risk of having collateral reactions that can cause damage to the hair and produce toxic intermediates and by-products is minimal. Hence, the use of a hair dye with fewer ingredients such as those based on the present invention helps to reduce damage to the hair.

## Other uses

- The present invention, comprising the synthesis of a metal complex in powder or colloidal form to be used as a catalyst for several reactions or polymerization, is characterized by the one-step production of an ecofriendly catalyst for reactions of polymerization and eco-friendliness. Transition metal β-diketonates may be used to substitute the toxic mercury or tin catalysts for the polyurethane formation such as titanium, copper.
- Use of metal complexes in all types of catalysis.
- Several uses in decorative design in the skin use in different tribes around the world. such as mehndi or sindoor.
- Oxidative hair dyeing.
- By using lanthanide complexes a broad variety of materials that can be applied in magnetic resonance imaging.
- Precursor for superconducting.
- Chelating agent of intermediate for other reactions.
- Dye or stain for several substrates such as paper, textile, leather, stone, wood, natural or synthetic polymers or fibres.
- Applications related with the optical properties in solar cells, nonlinear optics, display devices, optical data storage
- medicinal and biomedical applications including both imaging and therapy

## Example 1: Titanium complexes of curcumin

## Materials

Either curcumin for synthesis (Merck) or curcumin, 95% (total curcuminoid content) from Turmeric rhizome (Alfa Aesar)
Titanium(IV) n-butoxide, 99% (Acros Organics)

**X-ray diffraction (XRD)**

Diffractograms were obtained in a Xpert PANalytical Empyrean Serie II-alpha1, model 2012 with a K-alpha1 wavelength of 1.5405980 and K-Alpha2 wavelength of 1.5444260 (Cu) from $2\theta$ range from 5° to 50° with a scan step size of 0.026.

**Synthesis**

Titanium n-butoxide (0.3410 g, 1 mmol) was mixed with curcumin (0.3686, 1 mmol) in a agate mortar and immediately milled using a hand agate mortar and pestle. The reaction started with an orange-reddish paste that turned, after five minutes of grinding, into a red colored homogeneous powder. The reaction yielded a fine red powder with a yield of 88%. The complete yield is only diminished by the difficulty to remove the powder from the mortar. The product of the reaction is let to rest for one hour.

For X-ray diffraction analysis, the titanium-curcumin complex in powder form is dried at 124°C for 12 hour in order to remove water or n-butanol included in the composite.

**Discussion**

Small differences in the comparison between the diffraction patterns of the crystalline curcumin and the titanium-curcumin complexes such as at $2\theta \approx 13.5°$ reveal a new crystalline material that is different from the curcumin crystal structure.

**Example 2: Sunscreen with concealer effect**

**Materials**

Either curcumin for synthesis (Merck) or curcumin, 95% (total curcuminoid content) from Turmeric rhizome

(Alfa Aesar)

    Titanium(IV) n-butoxide, 99% (Acros Organics)
    2-4-Pentanedione, 99+% (Acros Organics)

**Synthesis**

Titanium n-butoxide (0.3678 g, 1 mmol) was mixed with curcumin (0.3362 g, 1 mmol), and to this mixture 2-4-Pentanedione (0.0747 g, 0.75 mmol) was added in a agate mortar and immediately milled using a hand agate mortar and pestle. The reaction started with an orange-reddish paste that turned, after five minutes of grinding, into a red colored homogeneous powder. The reaction yielded a fine red powder with a yield of 85%. The complete yield is only diminished by the difficulty to remove the powder from the mortar.

**Discussion**

The titanium-curcumin complex was added to the cetomacrogol and mixed by using a mechanical stirrer or a mortar. Only 1% weight of the previously prepared titanium curcumin complex in cetomacrogol crème provided a beautiful and stable purple color in the cream. The purple sunscreen with concealer effect form a tender and natural texture on the skin while masking without pasting. Although redundant, it is not white, therefore, does not have a chalky appearance.

Different hues of red color can be produced varying the amount of additive or replacing it for another one. The sun protection factor can be adjusted either by varying the amount of metal curcumin complexes or by the addition of other natural or synthetic filters.

**Example 3: Niobium-curcumin** complexes (not according to the claimed invention)

**Materials**

Either curcumin for synthesis (Merck) or curcumin, 95% (total curcuminoid content) from Turmeric rhizome (Alfa Aesar)
Niobium n-butoxide, 99% (metals basis) (Alfa Aesar)

**X-ray diffraction (XRD)**

Diffractograms were obtained in a Xpert PANalytical Empyrean Serie II-alpha1, model 2012 with a K-alpha1 wavelength of 1.5405980 and K-Alpha2 wavelength of 1.5444260 (Cu) from $2\theta$ range from 5° to 50° with a scan step size of 0.026.

**Synthesis**

Niobium n-butoxide (0.2539 g, 0.55 mmol) was mixed with curcumin (0.1844 g, 0.5 mmol) in a ceramic mortar and immediately milled using a hand agate mortar and pestle. The reaction started with a dark red sticky paste that turned, after two minutes of grinding, into a homogeneous powder of red color bordering black. The complete yield is only diminished by the difficulty to remove the sticky material from the mortar.

No additional treatment was carried out in the final niobium-curcumin complex for the XRD analysis.

**Discussion**

The diffraction pattern showed a semi-crystalline material, although there was no additional treatment to remove the possible water or n-butanol present by inclusion in the complex.

**Relevant background art**

López-Periago et al. (3 May 2017) Metal organic frameworks precipitated by reactive crystallization in supercritical CO2, Crystal Growth and Design, 17(5), 2864-2872 discloses the preparation of Zn3(CMC)2 from Zn(acac) (i.e. a zinc β-diketonate and not a metal alkoxide) and curcumin (i.e. CMC) by reactive crystallization in supercritical CO2 with 2 % of v/v ethanol. (abstract and section 3.3.3). No mechanochemical tool according to IUPAC was used.

US 2017/275309 A1 discloses in synthetic example 1, the synthesis of titanium diisopropoxibis (trimethoxysilylpropyl acetoacetate) (see typical average structure (formule (7)) by stirring titanium tetraisopropoxide (i.e., a metal alkoxide) and trimethoxysilylpropyl acetoacetate (i.e., a β-ketoester but neither β-diketone nor a polyphenol) in the absence of solvent. After performing stirring at room temperature for 24 h, isopropanol as by-product was distilled away (par. 0092 last sentence). No mechanochemical tool according to IUPAC was used.

CN 107 417 719 A discloses the preparation of titanium β-diketones in the absence of solvent. Diisopropoxy diacetylacetone titanium was obtained by stirring isopropyl titanate (a metal alkoxide) and acetylacetone (a β-diketone but not a polyphenol) and after removing isopropanol. No mechanochemical tool according to IUPAC was used.

US 2011/250126 A1 discloses in comparative example 1, the synthesis of a bis(isopropoxy)bis(methyl-4,4-dimethyl-3-oxopentanoato)titanium by stirring titanium tetrakis isopropoxide (i.e. a metal alkoxide) with methyl 4,4-dimethyl-3-oxopentanoate (i.e., a β-ketoester but neither a β-diketone nor a polyphenol) in the absence of solvent (par. 0074) forming a yellow viscous solution. No mechanochemical tool according to IUPAC was used.

US 2008/254216 A1 in Example B-5 discloses the solventless synthesis of tetrakis(2-methoxy-6 methyl-3,5-heptanodionato)titanium (IV) -Ti(mopd)4 in liquid form by stirring titanium (IV) tetraethoxide (i.e. a metal alkoxide) and 2-methoxy-6-methyl-3,5-hepatenedione (a β-diketone but not a polyphenol). The mixture was stirred for one hour for carrying out a reaction with distillation-off of ethanol. No mechanochemical tool according to IUPAC was used.

Rahman et al. "Optical properties and X-ray photoelectron spectroscopic study or pure and Pb-doped TiO 2 thin films", JOURNAL OF PHYSICS AND CHEMISTRY OF SOLIDS., vol. 60, no. 2, 1 January 1999, pages 201-210, in Experimental does not discloses directly the synthesis of a sol solution since the "details of the sol solution preparation for the pure TiO2 was described elsewhere" (Experimental page 203, first par., last sentence). "Elsewhere" is (ref 6 of Rahman et al 1996). Elsewhere in Experimental Details described the synthesis of a TiO2 sol-solution by sol-gel method using titanium tetra-isopropoxide (i.e. a metal alkoxide), acetylacetone (i.e. a β-diketone), water, ethanol, and nitric acid. Polyphenols were no used as reactants. No mechanochemical tool according to IUPAC was used.

Sevastyanov et al., "Low-temperature synthesis of nanodispersed titanium, zirconium, and hafnium carbides", RUSSIAN JOURNAL OF INORGANIC CHEMISTRY, NAUKA/INTERPERIODICA, MO, vol. 56, no. 5, 1 June 2011, pages 661-672, on page 667 discloses the titanium containing precursor which was synthesized by reacting titanium tetrabutoxide (i.e., a metal alkoxide) with acetylacetone (i.e., a β-diketone) (page 667, second par. Right). "the metal-containing solutions prepared in this way" - i.e. previous second par. right on page 667 - "were used for working out gel-formation process in the absence and in the presence of a polymeric carbon source" (page 667, third, par. first sentence). *"The*

*carbonaceous component used was a solution of a resol-type phenol/formaldehyde resin...in ethanol, since the resin provides a high carbon yield upon pyrolysis"* states on page 669, left, fourth parr. No polyphenol was used. No mechanochemical tool according to IUPAC was used.

Vigato et al., "The evolution of β-diketone or β-diketophenol ligands and related complexes", COORDINATION CHEMISTRY REVIEWS, ELSEVIER SCIENCE, AMSTERDAM, NL, vol. 253, no. 7-8, 1 April 2009, pages 1099-1201, is a review article presenting the evolution of β-diketone or β-diketophenol ligands and related complexes (see title). "The role of the different metal ions in directing the preparation pathways and tunning peculiar properties to the resulting systems is also discussed (see fist page, abstract, last sentence). The metal ions were from metal salts (see all references presented in the section 15). Metal alkoxide was not hinted at or suggested as a metal precursor. No mechanochemical tool according to IUPAC was used.

WO 2006/106366 A1 discloses an oxidative hair dye system comprises a hair dye (i.e., mixture of a base and a coupler (page 10 line, 13-30)), an organometallic compound (page 8-10) and an oxidising agent (page 11, line 11-14). The organometallic compound being a tetraalkyl titanate (i.e. a metal alkoxide) or a titanate chelate (i.e. a β-diketone o β-ketoester complex commercially obtained) were also useful for enhancing the substantivity of topical compositions applied to the body. (see abstract). Neither β-diketone nor polyphenol were used to produce the compositions. No mechanochemical tool according to IUPAC was used.

US 4 263 333 A discloses a complex and a process for preparing said complex formed by the reaction of curcumin, turmeric with metal ions. (abstract). The source of metal ions, usual a salt (page 3 line 3), is a stannous ion, a zinc ion, and mixture thereof (claim 1). Metal alkoxides were not used as metal precursors. It also discloses water soluble metal curcumin complex capable of producing varying hues of the same colors for use in foodstuffs (abstract). No mechanochemical tool according to IUPAC was used.

**Citations:**

1 Wanninger S., Lorenz, V., Subhan, A. & Edelmann, F. (2015). Metal complexes of curcumin synthetic strategies and medicinal applications. Chemical Society Reviews, 44(15), 4686-5002.

2 Kühlwein, F., Polborn, K., & Beck, W. (1997). Metallkomplexe von Farbstoffen. VIII Obergangsmetallkomplexe des Curcumins und seiner Derivate. Zeitschrift Für Anorganische Und Allgemeine Chemie. 623(8), 1211-1219.

3 Spicer, G., & Strickland, J. (1952). 906. Compounds of curcumin and boric acid. Part I. The structure of rosocyanin. Journal of the chemical society (Resumed), 4644-4650.

4 Spicer, G., & Strickland, J. (1952). 907. Compounds of curcumin and boric acid. Part II. The structure of rubrocurcumin. Journal of the chemical society (Resumed), 4650-4653.

5 Hayes, M. & Metcalfe, J. (1962). The boron-curcumin complex in the determination of trace amounts of boron. The Analyst, 87(1041), 956-969.

6 Tsuchikawa, M., Takao, A., Funaki, T., Sugihara, H., & Ono, K. (2017). Multifunctional organic dyes: Anion-sensing and light-harvesting properties of curcumin boron complex. RSC Advances, 7(58), 36612-36616.

7 Das, S., & Wei, Y. (2011). Immobilization of Enzymes in Sol-Gel Mesoporous Silica, Enzymatic Digestion of Biomass, and Silica-Curcumin Hybrid Materials. ProQuest Dissertations and Theses

8 Mehrotra, R. C., Bohra, R. & Gaur, D. P. (1978). Metal β-diketones and allied derivatives, Academic Press, London.

9 Bradley, D. C., Mehrotra, R. C. & Gaur, D. P. (1978). Metal alkoxides. Academic Press, London; Bradley, D. C., Mehrotra R. C., Rothwell I. P. and Singh A. (2001) Alkoxo and aryloxo derivatives of metals, Academic Press, London.

10 DE000001945303A

11 EP2421509B1

12 Parisi, O. I. et al. (2014). "Polyphenols and their formulations: differents strategies to overcome the drawbackas associated with their poor stability and bioavailability", Chapter 4. In: Polyphenols in Human Health and Disease, Vol.1,( Eds Watson, D., Preedy, V., Zibadi S.), 1st ed, 29-45, Academic Press, London.

[13] EP 0431755A1

[14] US5266344

[15]Tobin, D. J (ed) (2005) Hair in toxicology: an important bio-monitor, The Royal society of Chemistry publishing, Cambridge.

[16] Corbett, J. F. (1971) "Hair Dyes", Chapter VII, in: The Chemistry of Synthetic dyes, Vol. 5 (Ed Venkataraman K.) Academic Press. New York and London.

[17] Putzig D. E & Moncarz J. R. (2007). "Titanium compounds, Organic", in: Kirk-Ohmer Encyclopedia of Chemical Technology, Vol. 25, 5th ed., 71-88, John Wiley & Sons, New York.

[18] Pettinari C, Marchetti, F. Drozdov A. (2003). "β-diketones and related ligands", in: Comprenhensive coordination chemistry II, Vol. 1 (Eds McCleverty J. A. and Meyer T. J.), 2nd edn, Elsevier, Amsterdam, pp. 97-115.

[19] Quideau S., Deffieux D., Douat-Casassus C. & Pouységu L. (2011). Plant polyphenols: chemical properties, biological activities, and synthesis. Angew Chem Int Ed, 50(3): 586-621.

[20] Tsimogiannis D. & Oreopoulou V. (2018). Chapter 16 "Classification of phenolic compounds in plants",. in: Watson R. R. (Polyphenols in plants: isolation, purification and extract preparation, Academic Press, London, pp. 263-284.

[21] Harborne J. B. (Ed) (1989). Methods in plant biochemistry: 1. Plant phenolics, Academic Press, London; Watson R. R. (Ed) (2019). Polyphenols in plants:isolation, purification and extract preparation, Acedemic Press London.

[22] IUPAC, Compendium of chemical technology, 2nd ed. (the "Gold book"). Compiled by A. D, McNaught and A. Wilkinson (1997). Blackwell Scientific Publications, Oxford.

[23] Turova, N.Y., Turevskaya E.P., Kessler V.G. & Yanovskaya M.I. (2006). The Chemistry of Metal Alkoxides, Springer Science & Business Media, Dordrecht.

[24] Tanaka, K. (2003). Solvent-free organic synthesis, Wiley-VCH Verlag GmbH & Co. KGaA, Weinheim.

[25] Hernández J. G. & Bolm C. (2017). Altering product selectivity by mechanochemistry. J. Org. Chem., 82, 4007-4019.

**Claims**

1. Metal complex in powder form comprising the product of the reaction of

    (a) at least one polyphenol or of at least one β-diketone and at least one polyphenol with
    (b) at least one metal alkoxide

obtainable by mechanochemical synthesis by grinding or milling together reactant (a) and reactant (b), with less than 10 wt.% of solvents, based on the total weight of the reaction mixture, wherein the mechanochemical synthesis includes the use of a mechanical mortar or pestle, high speed milling, ball milling, attrition milling or planetary milling, wherein the at least one metal alkoxide (b) is a compound of formula $M(OR)_x$ or $[M(OR)_x]_n$ or heterometallic alkoxide or polymeric metal alkoxide or metal-oxo-alkoxide, wherein,

    - *M is* titanium, or one *M* of the heterometalic alkoxide is titanium,
    - *R* is an organic radical such as methoxide, ethoxide, propoxide (n- and iso-), butoxide (n-, iso-, sec-, and tert-), amiloxide (n-, sec-, tert-) and neopentyloxide, aryloxide,
    - *x* corresponds to the valency of the metal *M,*
    - n corresponds to the degree of molecular association.

2. Metal complex according to claim 1 further comprising
(c) at least one additive

3. Metal complex according to claim 1 to 2 wherein:

the at least one β-diketone compound or its tautomers is selected from:

- synthetic β-diketones either with modification of the methylene group or with the introduction of nitrogen or sulfur groups into the keto-enol moiety, selected from: acetylacetone (pentane-2,4-dione), benzoylacetone, dibenzoylmethane, diisobutyryl-methane, 2,2-dimethyl-heptane-3,5-dione, 2,2,6-trimethyl-heptane-3,5-dione, dipivaloyl-methane, 2-thenoylacetone, 2-furoylacetone, cycloheptane-1,3-dione, diferuloylmethane, thio-β-diketones, chiral β-diketones, synthetic derivatives of 1,7-bisphenyl heptane-3,5-diones, synthetic curcuminoids or curcumin derivatives, curcumin conjugates, tetrahydrocurcumin and mixtures thereof;
- naturally occurring β-diketones selected from: curcumin and its derivatives including curcumin species (Zingiberaceae): from turmeric rhizome; curcumin, demethoxycurcumin, bisdemethoxycurcumin or mixtures thereof; green turmeric, dried turmeric, turmeric oleoresin; curcumin metabolites including tetrahydrocurcumin, curcumin glucuronide; other curcuminoids including cyclocurcumin, cassumunin A, cassumunin B, cassumunin C and mixtures thereof; formulations containing curcumin including nanoparticles, liposomal encapsulation, phospholipid complexes, emulsions, capsules, tablets, and powders, either used alone or in combination with other compounds; and

the at least one polyphenol either synthetic or natural is selected from

- simple aglycone or glycone phenols, preferably arbutin;
- phenolic acids, preferably vanillic acid and syringic acid;
- phenolic aldehydes, preferably vanillin or syringaldehyde;
- phenylethanoids, preferably tyrosol;
- phenylpropanoids, preferably ferulic acid, p-coumaryl alcohol, cafeic acid and rosmarinic acid;
- stilbenoids, preferably resveratrol;
- anthraquinones and anthrones, preferably aloe emodin, alizarin, purpurin or carminic acid;
- xanthonoids, preferably mangiferin;
- naphthoquinones, preferably lawsone, alkannin or juglone;
- raspberry ketone;
- open bridge flavonoids, preferably selected from 1-(2-hydroxyphenyl)-3-phenyl-1,3-propanedione isolated from the root bark of Pongamia pinnata or the stem bark of Millettia ovalifolia;
- closed bridge flavonoids, preferably quercetin, chrysin, rutin, naringin, or hesperidin;
- diarylheptanoids, preferably curcumin;
- phenolic dimers and condensation oligomeric products, preferably selected fromellagic acid, lignans, biscoumarins, dimers and trimers of ferulic acid, cafeic acid oligomers, bis-xanthones and xantholignoids, dimeric or oligomeric stilbenoids, bisantraquinones, dianthrones, di and oligomeric stilbenes;
- polymeric phenols, preferably selected from gallotannins, ellagitannins, proanthocyanidines, phlorotannins, thearubigins, kraft lignin and lignosulfonates;
- hybrid phenolic, preferably selected from carvacrol, thymol, THC, CBG, carnosic acid, carnosol, cardanol, bilobol, gingerols, shogaols and ginkgolic acids;
- aqueous or organic extract or tinctures of polyphenols or mixtures of polyphenols.

4. Metal complex according to any of the preceding claims **characterized by** a metal alkoxide to β-diketone molar ratio of 100:1 to 1:100 and a metal alkoxide to polyphenol molar ratio of 100:1 to 1:100.

5. Metal complex according to any of the preceding claims
wherein the β-diketone is a naturally or synthetically occurring β-diketone, preferably curcumin, and/or the polyphenols are a natural or synthetic flavonoid, preferably quercetin, and/or a natural or synthetic phenylpropanoid, preferably ferulic acid, or a natural or synthetic phenolic dimer, preferably ellagic acid, and a natural or modified polymeric phenol, preferably lignin;

6. Process for the fabrication of a metal polyphenol complex or a metal complex from the reaction with a β-diketone and a polyphenol in powder form according to the claims 1 to 5, comprising:

i. mixing or adding the at least one metal alkoxide compound (b) with or to the at least one polyphenol or mixing the at least one metal alkoxide compound (b) to the at least one β-diketone compound and polyphenol (a) in a container in any order, simultaneously or sequentially;

ii. applying mechanochemical synthesis by grinding or milling the reactants until a homogeneous powder is obtained, wherein the mechanochemistry includes the use of a mechanical mortar and pestle, high speed milling, ball milling, attrition milling and planetary milling and

iii. optionally letting rest the powder,

wherein the at least one metal alkoxide (b) is a compound of formula $M(OR)_x$ or $[M(OR)_x]_n$ or heterometallic alkoxide or polymeric metal alkoxide or metal-oxo-alkoxide, wherein,

- *M is* titanium, or one *M* of the heterometalic alkoxide is titanium,
- *R* is an organic radical such as methoxide, ethoxide, propoxide (n- and iso-), butoxide (n-, iso-, sec-, and tert-), amiloxide (n-, sec-, tert-) and neopentyloxide, aryloxide,
- *x* corresponds to the valency of the metal *M*
- n corresponds to the degree of molecular association.

7. Process according to claim 6, wherein the container is a hand or mechanical mortar or pestle or a hand or comb, , and whereby an optional moderate increase of the temperature is applied.

8. Process according to claim 6 to 7 further comprising adding at least one additive compound (c) to at least one β-diketone and/or at least one polyphenol (a) and at least one metal alkoxide (b) simultaneously, or to every reactant (a) or (b), or after the reactants are put into contact or after the final complex is formed,

wherein the at least one metal alkoxide (b) is a compound of formula $M(OR)_x$ or $[M(OR)_x]_n$ or heterometalic alkoxide or polymeric metal alkoxide or metal-oxo-alkoxide and

- *M is* titanium, or one *M* of the heterometalic alkoxide is titanium,
- *R* is an organic radical such as methoxide, ethoxide, propoxide (n- and iso-), butoxide (n-, iso-, sec-, and tert-), amiloxide (n-, sec-, tert-) and neopentyloxide, aryloxide,
- *x* corresponds to the valency of the metal *M,*
- n corresponds to the degree of molecular association, and

said additive compound (c) is selected from:

the group of further β-diketones, further polyphenols, further metal β-diketones, further metal polyphenols, solvents, fatty oils, phenolic acids, fatty alcohols, organic acids, vitamins, aminoacids, lipids, proteins, carboxylic acids, synthetic or natural colorants, wetting agents, swelling agents, penetrants, pH regulators, surfactants, perfumes, thickeners, milling adjuvants, salts, oxides, water,

whereby the amount of additive compound is between 0.01 wt% and 50 wt% of the mixtures of compound(s) (a) and compound(s) (b), preferably 5-30 wt%.

9. Process according to claims 6-8, comprising

i. adding the metal alkoxide compound (b) to a curcumin and/or quercetin and/or ferulic acid and/or ellagic acid and/or lignin compound (a) and consecutively adding the compound (c) in a hand or mechanical mortar;

ii. applying mechanochemical synthesis by grinding or milling the reactants until a homogeneous powder is obtained;

iii. letting rest whereby the powder obtained is a colored material with colors such as red, yellow, orange, violet, pink, green or brown.

10. Composition in the form of an emulsion, miniemulsion, microemulsion, suspension or dispersion comprising a metal complex according to any of claims 1-5, preferably wherein the metal complex is encapsulated in a polymer matrix.

11. Cosmetic, sunscreen, pharmaceutical food, staining, painting or coating composition comprising a metal complex according to any of claims 1-5.

12. Process for the fabrication of a sunscreen product with tone concealer, to prevent premature aging of the keratinous material and to prevent skin or hair disorders, comprising the following steps:

(a) a metal complex according to any of claims 1-5 is mixed with a vehicle in such a way that the desired sun protection factor is obtained, whereby said vehicle is selected from: a cream base for preparing skin or hair creams without active ingredients, without perfumes, without parabens such as cetomacrogol crème commercially available, comprising water, decyl oleate, cetaryl alcohol, cetearth-20, sorbitol, sorbic acid, or any cetomacrogol crème; a pasty fatty substance such as waxes, gum or mixtures thereof, an oil, a fatty alcohol, a surfactant, a gel, a powder, a UV filter and/or UV absorber, a skin or hair protecting, an emollient, an humectant, an emulsifier, an skin or hair conditioning, a refatting agent, a masking agent, an emulsion stabilizer, a cleansing agent, an antioxidant, an opacifying agent, a solvent, a viscosity controller, a bulking agent, an abrasive, an anticaking agent, a preservative, a parfum, a buffer agent, an antimicrobial, a salt, water or mixtures thereof or any other cosmetically or pharmaceutically acceptable vehicle;

(b) optionally adjusting the desired sun protection factor by the addition of further synthetic or natural filters.

13. Use of at least one metal complex or mixture thereof according to any of claims 1-5 as a hair dye, textile dye, keratinous dye, wood dye, food dye, or pharmaceutical dye in powder form or as colloids; as catalyst for reactions and polymerization either alone or in combination with other dyes or catalysts such as zeolites.

**Patentansprüche**

1. Metallkomplex in Pulverform, der das Produkt der Reaktion von

(a) mindestens einem Polyphenol oder von mindestens einem β-Diketon und mindestens einem Polyphenol mit
(b) mindestens einem Metallalkoxid umfasst,

hergestellt durch mechanochemische Synthese durch Mahlen oder Vermahlen von Reaktant (a) und Reaktant (b), mit einem Lösungmittelanteil von weniger als 10 Gew.-% bezogen auf das Gesamtgewicht des Reaktionsgemisches, wobei die mechanochemische Synthese die Verwendung eines mechanischen Mörsers oder Stössels, einer Hochgeschwindigkeitsmühle, Kugelmühle, Reibfräse oder Planetenfräse umfasst, wobei das mindestens eine Metallalkoxid (b) eine Verbindung der Formel $M(OR)_x$ or $[M(OR)_x]_n$ oder ein heterometallisches Alkoxid oder polymeres Metallalkoxid oder Metall-Oxo-Alkoxid ist, wobei,

- $M$ Titan ist, oder ein $M$ des heterometallischen Alkoxids Titan ist;
- $R$ ein organischer Rest wie Methoxid, Ethoxid, Propoxid (n-und iso-), Butoxid (n-, iso-, sec-und tert-), Amyloxid (n-, sec-, tert-) und Neopentyloxid, Aryloxid ist;
- $x$ der Wertigkeit des Metalls $M$ entspricht,
- $n$ dem Grad der molekularen Assoziation entspricht.

2. Metallkomplex nach Anspruch 1, ferner umfassend
(c) mindestens ein Additiv

3. Metallkomplex nach Anspruch 1 bis 2, wobei:

die mindestens eine β-Diketonverbindung oder deren Tautomere ausgewählt ist aus

- synthetischen β-Diketonen, entweder mit Modifikation der Methylengruppe oder mit der Einbringung von Stickstoff- oder Schwefelgruppen in den Keto-Enol-Rest, ausgewählt aus: Acetylaceton (Pentan-2,4-dion), Benzoylaceton, Dibenzoylmethan, Diisobutyryl-methan, 2,2-Dimethyl-heptan-3,5-dion, 2,2,6-Trimethyl-heptan-3,5-dion, Dipivaloylmethan, 2-Thenoylaceton, 2-Furoylaceton, Cycloheptan-1,3-dion, Diferuloylmethan, Thio-β-Diketonen, chiralen β-Diketonen, synthetischen Derivaten von 1,7-Bisphenylheptan-3,5-dionen, synthetischen Curcuminoiden oder Curcumin-Derivaten, Curcumin-Konjugaten, Tetrahydrocurcumin und Mischungen davon;
- natürlich vorkommenden β-Diketonen, ausgewählt aus Curcumin und seinen Derivaten, einschließlich Curcumin-Spezies (Zingiberaceae): aus Turmeric-Rhizom; Curcumin, Demethoxycurcumin, Bisdemethoxycurcumin oder Mischungen davon; grüne Gelbwurz, getrocknete Gelbwurz, Kurkuma-Oleoresin; Curcumin-Metaboliten, einschließlich Tetrahydrocurcumin, Curcuminglucuronid; andere Curcuminoide, einschließlich Cyclocurcumin, Cassumunin A, Cassumunin B, Cassumunin C und Mischungen davon; Formulierungen, die Curcumin enthalten, einschließlich Nanopartikel, liposomale Verkapselung, Phospholipid-Komplexe, Emulsionen, Kapseln, Tabletten und Pulver, entweder allein oder in Kombination mit anderen Verbindungen; und

das mindestens eine Polyphenol, entweder synthetisch oder natürlich, ausgewählt ist aus

- einfachen Aglykon- oder Glyconophenolen, vorzugsweise Arbutin;
- Phenolsäuren, vorzugsweise Vanillinsäure und Syringainsäure;
- Phenolische Aldehyden, vorzugsweise Vanillin oder Syringaldehyd;
- Phenylethanoloiden, vorzugsweise Tyrosol;
- Phenylpropanoiden, vorzugsweise Ferulasäure, p-Cumarylalkohol, Cafeisäure und Rosmarinsäure;
- Stilbenen, vorzugsweise Resveratrol;
- Anthrachinonen und Anthronen, vorzugsweise Aloe Emodin, Alizarin, Purinpurin oder Carminsäure;
- Xanthonoiden, vorzugsweise Mangiferin;
- Naphthochinonen, vorzugsweise Lawsone, Alkannin oder Juglon;
- Himbeerketon;
- Offene-Brücken-Flavonoiden, vorzugsweise ausgewählt aus 1-(2-Hydroxyphenyl)-3-phenyl-1,3-propandion, isoliert aus der Wurzelrinde von Pongamia pinnata oder der Stammrinde von Milchitia ovalifolia;
- Geschlossene-Brücken-Flavonoiden, vorzugsweise Quercetin, Chrysin, Rutin, Naringin oder Hesperidin;
- Diarylheptanoiden, vorzugsweise Curcumin;
- phenolische Dimeren und kondensationsoligomerischen Produkten, vorzugsweise ausgewählt aus Ellagsäure, Lignans, Biscumarinen, Dimeren und Trimeren von Ferulasäure, Cafeisäure-Oligomeren, Bis-Xanthonen und Xantholignoiden, dimerischen oder oligomerischen Stilbenoiden, Bisantrachinonen, Dianthronen, di- und oligomerischen Stilbenen;
- polymerischen Phenolen, vorzugsweise ausgewählt aus Gallotanninen, Ellagitanninen, Proanthocyanidinen, Phlorotanninen, Thearubiginen, Kraftlignin und Ligninsulfonaten;
- Hybrid-Phenol, vorzugsweise ausgewählt aus Carvacrol, Thymol, THC, CBG, Carnosic-Säure, Carnosol, Cardanol, Biloboll, Galeriolen, Shogaolen und ginkgolischen Säuren;
- wässrigem oder organischem Extrakt oder Tinkturen von Polyphenolen oder Mischungen von Polyphenolen.

4. Metallkomplex nach einem der vorangehenden Ansprüchen, **gekennzeichnet durch** ein Molverhältnis Metallalkoxid zu β-Diketon zwischen 100:1 und 1:100, sowie ein Molverhältnis Metallalkoxid zu Polyphenol zwischen 100:1 und 1:100.

5. Metallkomplex nach einem der vorhergehenden Ansprüche,
wobei das β-Diketon ein natürlich oder synthetisch auftretendes β-Diketon, vorzugsweise Curcumin, ist, und/oder die Polyphenole ein natürliches oder synthetisches Flavonoid, vorzugsweise Quercetin, und/oder ein natürliches oder synthetisches Phenylpropanoid, vorzugsweise Ferulasäure, oder ein natürliches oder synthetisches phenolisches Dimer, vorzugsweise Ellagsäure, und ein natürliches oder modifiziertes polymerisches Phenol, vorzugsweise Lignin, sind;

6. Verfahren zur Herstellung eines Metallpolyphenolkomplexes oder eines Metallkomplexes aus der Umsetzung mit einem β-Diketon und einem Polyphenol in Pulverform nach den Ansprüchen 1 bis 5, umfassend:

i. das Mischen oder Hinzufügen der mindestens einen Metallalkoxidverbindung (b) mit oder zu dem mindestens einen Polyphenol, oder das Mischen der mindestens einen Metallalkoxidverbindung (b) mit der mindestens einen β-Diketonverbindung und Polyphenol (a) in einem Behälter in beliebiger Reihenfolge, gleichzeitig oder nacheinander;
ii. die Anwendung einer mechanochemischen Synthese durch Mahlen oder Vermahlen der Reaktanten bis zum Erhalt eines homogenen Pulvers, wobei die Mechanochemie die Verwendung eines mechanischen Mörsers und Stössels, Hochgeschwindigkeitsmühlen, Kugelmühlen, Reibfräsen und Planetenfräsen umfasst, und
iii. gegebenenfalls das Ruhenlassen des Pulvers,

wobei das mindestens eine Metallalkoxid (b) eine Verbindung der Formel $M(OR)_x$ or $[M(OR)_x]_n$ oder heterometallisches Alkoxid oder polymeres Metallalkoxid oder Metall-Oxo-Alkoxid ist, wobei

- *M* Titan ist oder ein *M* des Heterometallalkoxids Titan ist,
- R ein organischer Rest wie Methoxid, Ethoxid, Propoxid (n-und iso-), Butoxid (n-, iso-, sec-und tert-), Amyloxid (n-, sec-, tert-) und Neopentyloxid, Aryloxid ist,

- x der Wertigkeit des Metalls M entspricht,
- n dem Grad der molekularen Assoziation entspricht.

7. Verfahren nach Anspruch 6, wobei der Behälter eine Hand oder ein mechanischer Mörser oder Stössel oder eine Hand oder ein Kamm ist, und wobei eine optionale mässige Erhöhung der Temperatur angewendet wird.

8. Verfahren nach Anspruch 6 bis 7, das ferner die Zugabe von mindestens einer Additivverbindung (c) zu dem mindestens einem β-Diketon und/oder mindestens einem Polyphenol (a) und mindestens einem Metallalkoxid (b) umfasst, gleichzeitig oder zu jedem Reaktionspartner (a) oder (b), oder nachdem die Reaktanten in Kontakt gebracht werden oder nachdem der Endkomplex gebildet wird,
wobei das mindestens eine Metallalkoxid (b) eine Verbindung der Formel $M(OR)_x$ or $[M(OR)_x]_n$ oder Heterometallalkoxid oder polymerisches Metallalkoxid oder Metall-Oxo-Alkoxid ist, und

- M Titan ist, oder ein M des Heterometallalkoxids Titan ist,
- R ein organischer Rest wie Methoxid, Ethoxid, Propoxid (n-und iso-), Butoxid (n-, iso-, sec-und tert-), Amyloxid (n-, sec-, tert-) und Neopentyloxid, Aryloxid ist,
- x der Wertigkeit des Metalls M entspricht,
- n dem Grad der molekularen Assoziation entspricht, und

die Additivverbindung (c) ausgewählt ist aus:

der Gruppe weiterer β-Diketone, weiterer Polyphenole, weiterer Metall-β-Diketone, weiterer Metallpolyphenole, Lösungsmittel, Fettöle, Phenolsäuren, Fettalkohole, organischen Säuren, Vitaminen, Aminosäuren, Lipiden, Proteinen, Carbonsäuren, synthetischen oder natürlichen Farbstoffen, Netzmitteln, Quellmitteln, Penetrationsmitteln, pH-Regulatoren, Tensiden, Duftstoffen, Verdickungsmitteln, Mahlhilfsstoffen, Salzen, Oxiden, wobei die Menge der Additivverbindung zwischen 0,01 Gew.-% und 50 Gew.-% der Mischungen aus Verbindung(en) (a) und Verbindung(en) (b), vorzugsweise 5-30 Gew.-%, beträgt.

9. Verfahren nach den Ansprüchen 6-8, beinhaltend

i. die Zugabe der Metallalkoxidverbindung (b) zu einem Curcumin und/oder Quercetin und/oder Ferulasäure und/oder E ' llagsäure und/oder Ligninverbindung (a) und nachfolgender Zugabe der Verbindung (c) in einer Hand oder einem mechanischen Mörser;
ii. Aufbringen einer mechanochemischen Synthese durch Mahlen oder Vermahlen der Reaktanten bis zum Erhalt eines homogenen Pulvers;
iii. Ruhenlassen, wobei das erzeugte Pulver ein farbiges Material unter anderen in den Farben Rot, Gelb, Orange, Violett, Pink, Grün oder Braun ist.

10. Zusammensetzung in Form einer Emulsion, Miniemulsion, Mikroemulsion, Suspension oder Dispersion, die einen Metallkomplex nach einem der Ansprüche 1-5 umfasst, wobei der Metallkomplex vorzugsweise in einer Polymermatrix verkapselt ist.

11. Kosmetische, Sonnenschutz-, pharmazeutische Nahrungsmittel-, Färbe-, Lackierungs- oder Beschichtungszusammensetzung, die einen Metallkomplex nach einem der Ansprüche 1-5 umfasst.

12. Verfahren zur Herstellung eines Sonnenschutzmittels mit getöntem Concealer zur Verhinderung der vorzeitigen Alterung des keratinischen Materials und Haut- oder Haarstörungen, das folgende Schritte umfasst:

(a) Ein Metallkomplex nach einem der Ansprüche 1-5 wird mit einem Trägerstoff so gemischt, dass der gewünschte Sonnenschutzfaktor erreicht wird, wobei der genannte Trägerstoff ausgewählt ist aus: einer Cremebasis zur Herstellung von Haut- oder Haarcremes ohne Wirkstoffe, ohne Parfums, ohne Parabene wie Cetomacrogol-Crème, kommerziell erhältlich, zusammengesetzt aus Wasser, Decyloleat, Cetyl-Alkohol, Cetearth-20, Sorbitol, Sorbinsäure, oder einer beliebige Cetomacrogol-Crème; einer pastösen Fettsubstanz, wie Wachse, Gummi oder Mischungen davon, einem Öl, einem Fettalkohol, einem Tensid, einem Gel, einem Pulver, einem UV-Filter und/oder UV-Absorber, einem Haut- oder Haarschutz, einem Weichmacher, einem Feuchthaltemittel, einem Emulgator, einer Haut- oder Haarkonditionierung, einem Rückfettungsmittel, einem Lösungsmittel, einem Viskositätsregler, einem Füllmittel, einem Antioxidans, einem Antibackmittel, einem Konservierungsmittel, einem Parfum, einem Puffermittel, einem antimikrobiellen Mittel, einem Salz, Wasser

EP 3 807 287 B1

oder Mischungen davon oder einem beliebigen anderen kosmetisch oder pharmazeutisch vertretbaren Vehikel;
(b) Gegebenenfalls wird der gewünschte Sonnenschutzfaktor durch Zugabe weiterer synthetischer oder natürlicher Filter eingestellt.

**13.** Verwendung mindestens eines Metallkomplexes oder einer Mischung davon nach einem der Ansprüche 1-5 als Haarfärbestoff, Textilfarbstoff, keratinischem Farbstoff, Holzfarbstoff, Lebensmittelfarbstoff oder pharmazeutischem Farbstoff in Pulverform oder als Kolloide; als Katalysator für Reaktionen und Polymerisation entweder allein oder in Kombination mit anderen Farbstoffen oder Katalysatoren wie Zeolithen.

**Revendications**

1. Complexe métallique sous forme de poudre comprenant le produit de la réaction

   (a) d'au moins un polyphénol ou d'au moins une β-dicétone et d'au moins un polyphénol avec
   (b) au moins un alcoxyde métallique

   pouvant être obtenu par synthèse mécanochimique par pulvérisation ou broyage du réactif (a) et du réactif (b), avec moins de 10 % en poids de solvants, par rapport au poids total du mélange réactionnel, sachant que la synthèse mécanochimique comprend l'utilisation d'un mortier ou d'un pilon mécanique, le broyage à grande vitesse, le broyage à boulets, le broyage par attrition ou le broyage planétaire,
   sachant que ledit au moins un alcoxyde métallique (b) est un composé de formule $M(OR)_x$ ou $[M(OR)_x]_n$ ou un alcoxyde hétérométallique ou un alcoxyde métallique polymère ou un oxo-alcoxyde métallique, sachant que

   - $M$ est du titane, ou un $M$ de l'alcoxyde hétérométallique est du titane,
   - $R$ est un radical organique tel que méthoxyde, éthoxyde, propoxyde (n- et iso-), butoxyde (n-, iso-, sec- et tert-), amiloxyde (n-, sec-, tert-) et néopentyloxyde, aryloxyde,
   - $x$ correspond à la valence du métal $M$,
   - $n$ correspond au degré d'association moléculaire.

2. Complexe métallique selon la revendication 1, comprenant également
   (c) au moins un additif

3. Complexe métallique selon les revendications 1 à 2, sachant que :

   ledit au moins un composé β-dicétone ou ses tautomères sont sélectionnés parmi les éléments suivants :

   - β-dicétones synthétiques avec modification du groupe méthylène ou avec introduction de groupes azote ou soufre dans la fraction céto-énol, sélectionnées parmi les éléments suivants : acétylacétone (pentane-2,4-dione), benzoylacétone, dibenzoylméthane, diisobutyryl-méthane, 2,2-diméthyl-heptane-3,5-dione, 2,2,6-triméthyl-heptane-3,5-dione, dipivaloyl-méthane, 2-thénoyl-acétone, 2-furoyl-acétone, cycloheptane-1,3-dione, diféruloyl-méthane, thio-β-dicétones, chiral β-dicétones, dérivés synthétiques de 1,7-bisphényl heptane-3,5-diones, curcuminoïdes synthétiques ou dérivés de curcumine, conjugués de curcumine, tétrahydrocurcumine et leurs mélanges ;
   - β-dicétones naturelles sélectionnées parmi les éléments suivants : curcumine et ses dérivés, y compris les espèces de curcumine (Zingiberaceae) : à partir de rhizome de curcuma ; curcumine, déméthoxycurcumine, bisdéméthoxycurcumine ou leurs mélanges ; curcuma vert, curcuma séché, oléorésine de curcuma ; métabolites de curcumine y compris tétrahydrocurcumine, glucunoride de curcumine ; autres curcuminoïdes y compris cyclocurcumine, cassumunine A, cassumunine B, cassumunine C, et leurs mélanges ; formules contenant de la curcumine y compris nanoparticules, encapsulation liposomale, complexes phospholipidiques, émulsions, capsules, comprimés et poudres, soit utilisés soit seuls soit combinés à d'autres composés , et

   ledit au moins un polyphénol soit synthétique soit naturel est sélectionné parmi les éléments suivants :

   - phénols simples d'aglycone ou de glycone, de préférence arbutine ;
   - acides phénoliques, de préférence l'acide vanillique et l'acide syringique ;
   - aldéhydes phénoliques, de préférence vanilline ou syringaldéhyde ;

- phényléthanoïdes, de préférence tyrosol ;
- phénylpropanoïdes, de préférence acide férulique, alcool p-coumarylique, acide caféique et acide rosmarinique ;
- stilbénoïdes, de préférence resvératrol ;
- anthraquinones et anthrones, de préférence l'aloé-émodine, l'alizarine, la purpurine ou l'acide carminique ;
- xanthonoïdes, de préférence mangiférine ;
- naphtoquinones, de préférence lawsone, alcanine ou juglone ;
- cétone de framboise ;
- flavonoïdes à chaîne ouverte, sélectionnés de préférence parmi la 1-(2-hydroxyphényl)-3-phényl-1,3-propanedione isolée de l'écorce de racine de Pongamia pinnata ou de l'écorce de tige de Millettia ovalifolia ;
- flavonoïdes à chaîne fermée, de préférence quercétine, chrysine, rutine, naringine ou hespéridine ;
- diarylheptanoïdes, de préférence curcumine ;
- dimères phénoliques et produits oligomères de condensation, sélectionnés de préférence parmi l'acide ellagique, les lignanes, les biscoumarines, les dimères et trimères de l'acide férulique, les oligomères de l'acide caféique, les bis-xanthones et les xantholignoïdes, les stilbénoïdes dimères ou oligomères, les bisantraquinones, les dianthrones, les stilbènes dimères et oligomères ;
- phénols polymères, sélectionnés de préférence parmi les gallotanins, les ellagitanins, les proanthocyanidines, les phlorotanins, les théarubigines, la lignine Kraft et les lignosulfonates ;
- phénolique hybride, sélectionné de préférence parmi le carvacrol, le thymol, le THC, le CBG, l'acide carnosique, le carnosol, le cardanol, le bilobol, les gingérols, les shogaols et les acides ginkgoliques ;
- extrait aqueux ou organique ou teintures de polyphénols ou mélanges de polyphénols.

4. Complexe métallique selon l'une des revendications précédentes, **caractérisé par** un rapport molaire de l'alcoxyde métallique à la β-dicétone de 100:1 à 1:100 et un rapport molaire de l'alcoxyde métallique au polyphénol de 100:1 à 1:100.

5. Complexe métallique selon l'une des revendications précédentes,
sachant que la β-dicétone est une β-dicétone d'origine naturelle ou synthétique, de préférence la curcumine, et/ou les polyphénols sont un flavonoïde naturel ou synthétique, de préférence la quercétine, et/ou un phénylpropanoïde naturel ou synthétique, de préférence l'acide férulique, ou un dimère phénolique naturel ou synthétique, de préférence l'acide ellagique, et un phénol polymère naturel ou modifié, de préférence la lignine ;

6. Procédé de fabrication d'un complexe métallique de polyphénol ou d'un complexe métallique à partir de la réaction avec une β-dicétone et un polyphénol sous forme de poudre selon les revendications 1 à 5, comprenant les opérations suivantes :

   i. mélange ou ajout dudit au moins un composé alcoxyde métallique (b) avec ledit au moins un polyphénol ou mélange dudit au moins un composé alcoxyde métallique (b) audit au moins un composé β-dicétone et au polyphénol (a) dans un récipient dans un ordre quelconque, simultanément ou séquentiellement ;
   ii. application d'une synthèse mécanochimique par pulvérisation ou broyage des réactifs jusqu'à obtention d'une poudre homogène, sachant que la mécanochimie comprend l'utilisation d'un mortier mécanique et d'un pilon, le broyage à grande vitesse, le broyage à boulets, le broyage par attrition et le broyage planétaire et
   iii. en laissant éventuellement reposer la poudre,

sachant que ledit au moins un alcoxyde métallique (b) est un composé de formule $M(OR)_x$ ou $[M(OR)_x]_n$ ou alcoxyde hétérométallique ou alcoxyde métallique polymère ou oxo-alcoxyde métallique, sachant que

   - $M$ est du titane, ou un $M$ de l'alcoxyde hétérométallique est du titane,
   - R est un radical organique tel que méthoxyde, éthoxyde, propoxyde (n- et iso-), butoxyde (n-, iso-, sec- et tert-), amiloxyde (n-, sec-, tert-) et néopentyloxyde, aryloxyde,
   - $x$ correspond à la valence du métal $M$
   - n correspond au degré d'association moléculaire.

7. Procédé selon la revendication 6, sachant que le contenant est un mortier ou pilon manuel ou mécanique ou une main ou un peigne, et sachant qu'une augmentation modérée facultative de la température est appliquée.

8. Procédé selon les revendications 6 à 7, comprenant en outre l'ajout d'au moins un composé additif (c) à au moins une β-dicétone et/ou au moins un polyphénol (a) et au moins un alcoxyde métallique (b) simultanément, ou à chaque réactif (a) ou (b), ou après la mise en contact des réactifs ou après la formation du complexe final,
sachant que ledit au moins un alcoxyde métallique (b) est un composé de formule $M(OR)_x$ ou $[M(OR)_x]_n$ ou alcoxyde hétérométallique ou alcoxyde métallique polymère ou oxo-alcoxyde métallique et

- $M$ est du titane, ou un $M$ de l'alcoxyde hétérométallique est du titane,
- $R$ est un radical organique tel que méthoxyde, éthoxyde, propoxyde (n- et iso-), butoxyde (n-, iso-, sec- et tert-), amiloxyde (n-, sec-, tert-) et néopentyloxyde, aryloxyde,
- $x$ correspond à la valence du métal $M$,
- n correspond au degré d'association moléculaire, et

ledit composé additif (c) est sélectionné parmi :

le groupe des autres β-dicétones, autres polyphénols, autres β-dicétones métalliques, autres polyphénols métalliques, solvants, huiles grasses, acides phénoliques, alcools gras, acides organiques, vitamines, acides aminés, lipides, protéines, acides carboxyliques, colorants synthétiques ou naturels, agents mouillants, agents gonflants, agents pénétrants, régulateurs de pH, tensioactifs, parfums, épaississants, adjuvants de broyage, sels, oxydes, eau,
sachant que la quantité de composé additif est comprise entre 0,01 % en poids et 50 % en poids des mélanges de composé(s) (a) et de composé(s) (b), de préférence entre 5-30 % en poids.

9. Procédé selon les revendications 6 à 8, comprenant les opérations suivantes

i. ajout du composé alcoxyde métallique (b) à une curcumine et/ou quercétine et/ou à de l'acide férulique et/ou de l'acide ellagique et/ou à un composé ligninique (a) et ajout consécutif du composé (c) dans un mortier manuel ou mécanique ;
ii. application de la synthèse mécanochimique par pulvérisation ou broyage des réactifs jusqu'à obtention d'une poudre homogène ;
iii. maintien au repos, sachant que la poudre obtenue est un matériau coloré présentant des couleurs telles que rouge, jaune, orange, violet, rose, vert ou marron.

10. Composition sous la forme d'une émulsion, d'une miniémulsion, d'une microémulsion, d'une suspension ou d'une dispersion comprenant un complexe métallique selon l'une des revendications 1 à 5, de préférence dans laquelle le complexe métallique est encapsulé dans une matrice polymère.

11. Composition cosmétique, antisolaire, alimentaire pharmaceutique, de coloration, de peinture ou de revêtement comprenant un complexe métallique selon l'une des revendications 1 à 5.

12. Procédé de fabrication d'un produit de protection solaire avec un correcteur de teinte, pour empêcher le vieillissement prématuré de la matière kératinique et pour prévenir les troubles de la peau ou des cheveux, comprenant les étapes suivantes :

(a) un complexe métallique selon l'une des revendications 1 à 5 est mélangé avec un excipient de telle sorte que le facteur de protection solaire désiré est obtenu, sachant que
ledit excipient est sélectionné parmi les éléments suivants : une base de crème pour la préparation de crèmes pour la peau ou les cheveux sans principes actifs, sans parfums, sans parabènes telles que la crème au cétomacrogol disponible dans le commerce, comprenant de l'eau, de l'oléate de décyle, de l'alcool cétarylique, de la ceteareth-20, du sorbitol, de l'acide sorbique, ou toute crème au cétomacrogol ; une substance grasse pâteuse telle que les cires, la gomme ou leurs mélanges, une huile, un alcool gras, un surfactant, un gel, une poudre, un filtre UV et/ou un absorbeur d'UV, un protecteur de la peau ou des cheveux, un émollient, un humectant, un émulsifiant, un conditionneur de la peau ou des cheveux, un agent regraissant, un agent masquant, un stabilisateur d'émulsion, un agent nettoyant, un antioxydant, un agent opacifiant, un solvant, un contrôleur de viscosité, un agent gonflant, un abrasif, un antiagglomérant, un conservateur, un parfum, un agent tampon, un antimicrobien, un sel, de l'eau ou leurs mélanges ou tout autre excipient cosmétiquement ou pharmaceutiquement acceptable ;
(b) réglage facultatif du facteur de protection solaire souhaité par l'ajout d'autres filtres synthétiques ou naturels.

13. Utilisation d'au moins un complexe métallique ou d'un mélange de celui-ci selon l'une des revendications 1 à 5 comme colorant capillaire, colorant textile, colorant kératinique, colorant pour bois, colorant alimentaire ou colorant pharmaceutique sous forme de poudre ou de colloïdes ; comme catalyseur de réactions et de polymérisation soit seul soit en combinaison avec d'autres colorants ou catalyseurs tels que les zéolithes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 000001945303 A
- US 20080254216 A1
- EP 2421509 B1
- EP 0431755 A1
- US 5266344 A
- US 3506389 A
- GB 1025916 A

- WO 2006106366 A1
- US 5756610 A
- US 2017275309 A1
- CN 107417719 A
- US 2011250126 A1
- US 2008254216 A1
- US 4263333 A

**Non-patent literature cited in the description**

- Titanium compounds, Organic. Kirk-Ohmer Encyclopedia of chemical technology, vol. 25
- Kirk-Othmer Encyclopedia of Chemical Technology, vol. 2
- **LÓPEZ-PERIAGO et al.** Metal organic frameworks precipitated by reactive crystallization in supercritical CO2. *Crystal Growth and Design*, 03 May 2017, vol. 17 (5), 2864-2872
- **RAHMAN et al.** Optical properties and X-ray photoelectron spectroscopic study or pure and Pb-doped TiO 2 thin films. *JOURNAL OF PHYSICS AND CHEMISTRY OF SOLIDS.*, 01 January 1999, vol. 60 (2), 201-210
- **RAHMAN et al.** *Elsewhere*, 1996
- **SEVASTYANOV et al.** Low-temperature synthesis of nanodispersed titanium, zirconium, and hafnium carbides. *RUSSIAN JOURNAL OF INORGANIC CHEMISTRY, NAUKA/INTERPERIODICA, MO*, 01 June 2011, vol. 56, 661-672
- The evolution of β-diketone or β-diketophenol ligands and related complexes. **VIGATO et al.** COORDINATION CHEMISTRY REVIEWS. ELSEVIER SCIENCE, 01 April 2009, vol. 253, 1099-1201
- **WANNINGER S** ; **LORENZ, V.** ; **SUBHAN, A.** ; **EDELMANN, F.** Metal complexes of curcumin synthetic strategies and medicinal applications. *Chemical Society Reviews*, 2015, vol. 44 (15), 4686-5002
- **KÜHLWEIN, F** ; **POLBORN, K.** ; **BECK, W.** Metallkomplexe von Farbstoffen. VIII Obergangsmetallkomplexe des Curcumins und seiner Derivate.. *Zeitschrift Für Anorganische Und Allgemeine Chemie.*, 1997, vol. 623 (8), 1211-1219
- **SPICER, G.** ; **STRICKLAND, J.** 906. Compounds of curcumin and boric acid. Part I. The structure of rosocyanin.. *Journal of the chemical society (Resumed)*, 1952, 4644-4650

- **SPICER, G.** ; **STRICKLAND, J.** 907. Compounds of curcumin and boric acid. Part II. The structure of rubrocurcumin.. *Journal of the chemical society (Resumed)*, 1952, 4650-4653
- **HAYES, M.** ; **METCALFE, J.** The boron-curcumin complex in the determination of trace amounts of boron. *The Analyst*, 1962, vol. 87 (1041), 956-969
- **TSUCHIKAWA, M.** ; **TAKAO, A.** ; **FUNAKI, T.** ; **SUGIHARA, H.** ; **ONO, K.** Multifunctional organic dyes: Anion-sensing and light-harvesting properties of curcumin boron complex. *RSC Advances*, 2017, vol. 7 (58), 36612-36616
- **DAS, S.** ; **WEI, Y.** Immobilization of Enzymes in Sol-Gel Mesoporous Silica, Enzymatic Digestion of Biomass, and Silica-Curcumin Hybrid Materials. *ProQuest Dissertations and Theses*, 2011
- **MEHROTRA, R. C.** ; **BOHRA, R.** ; **GAUR, D. P.** Metal β-diketones and allied derivatives. Academic Press, 1978
- **BRADLEY, D. C.** ; **MEHROTRA, R. C.** ; **GAUR, D. P.** Metal alkoxides.. Academic Press, 1978
- **BRADLEY, D. C.** ; **MEHROTRA R. C.** ; **ROTHWELL I. P.** ; **SINGH A.** Alkoxo and aryloxo derivatives of metals. Academic Press, 2001
- Polyphenols and their formulations: differents strategies to overcome the drawbackas associated with their poor stability and bioavailability. **PARISI, O. I. et al.** Polyphenols in Human Health and Disease. Academic Press, 2014, vol. 1, 29-45
- Hair in toxicology: an important bio-monitor. The Royal society of Chemistry publishing, 2005
- Hair Dyes. **CORBETT, J. F.** The Chemistry of Synthetic dyes. Academic Press, 1971, vol. 5
- Titanium compounds, Organic. **PUTZIG D. E** ; **MONCARZ J. R.** Kirk-Ohmer Encyclopedia of Chemical Technology. John Wiley & Sons, 2007, vol. 25, 71-88

- β-diketones and related ligands. **PETTINARI C** ; **MARCHETTI, F.** ; **DROZDOV A.** Comprenhensive coordination chemistry II. Elsevier, 2003, vol. 1, 97-115
- **QUIDEAU S.** ; **DEFFIEUX D.** ; **DOUAT-CASASSUS C.** ; **POUYSÉGU L.** Plant polyphenols: chemical properties, biological activities, and synthesis.. *Angew Chem Int Ed*, 2011, vol. 50 (3), 586-621
- Classification of phenolic compounds in plants. **TSIMOGIANNIS D.** ; **OREOPOULOU V.** Polyphenols in plants: isolation, purification and extract preparation. Academic Press, 2018, 263-284
- Plant phenolics. Methods in plant biochemistry. Academic Press, 1989, vol. 1.
- Polyphenols in plants:isolation, purification and extract preparation. Acedemic Press, 2019
- **A. D, MCNAUGHT** ; **A. WILKINSON**. Compendium of chemical technology. Blackwell Scientific Publications, 1997
- **TUROVA, N.Y.** ; **TUREVSKAYA E.P.** ; **KESSLER V.G.** ; **YANOVSKAYA M.I.** The Chemistry of Metal Alkoxides. Springer Science & Business Media, 2006
- **TANAKA, K.** Solvent-free organic synthesis. Wiley-VCH Verlag GmbH & Co. KGaA, 2003
- **HERNÁNDEZ J. G.** ; **BOLM C.** Altering product selectivity by mechanochemistry.. *J. Org. Chem.*, 2017, vol. 82, 4007-4019